(19) Europäisches Patentamt
European Patent Office
Office européen des brevets

(11) Veröffentlichungsnummer: **0 240 680 A1**

(12) # EUROPÄISCHE PATENTANMELDUNG

(21) Anmeldenummer: **87102529.2**

(22) Anmeldetag: **23.02.87**

(51) Int.Cl.[3]: **C 07 D 277/10**
C 07 D 277/22, C 07 D 279/06
C 07 D 417/12, A 61 K 31/425
A 61 K 31/54

(30) Priorität: **08.03.86 DE 3607669**
**20.09.86 DE 3632042**

(43) Veröffentlichungstag der Anmeldung:
**14.10.87 Patentblatt 87/42**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Raddatz, Siegfried, Dr.**
**Jakob-Böhme-Strasse 21**
**D-5000 Köln 80(DE)**

(72) Erfinder: **Plümpe, Hans, Dr.**
**Kirschbaumstrasse 9**
**D-5600 Wuppertal 1(DE)**

(72) Erfinder: **Fruchtmann, Romanis**
**Konrad-Adenaur-Ufer 79-81**
**D-5000 Köln 1(DE)**

(72) Erfinder: **Kohlsdorfer, Christian, Dr.**
**Franz-Stryck-strasse 16**
**D-5042 Erftstadt(DE)**

(72) Erfinder: **Pelster, Bernhard, Dr.**
**Pleisufer 6a**
**D-5205 St- Augustin 1(DE)**

(54) **Diarylsulfidderivate.**

(57) Die neuen Diarylsulfidderivate können durch Umsetzung von Halogenverbindungen mit Thioharnstoffen oder Thiazolen mit Aminen oder Thiophenolen mit Halogenarylen oder Aminoethanolen mit Senfölen oder Halogensenfölen mit Aminen oder Dihalogenverbindungen mit substituierten Thioharnstoffen hergestellt werden. Die neuen Verbindungen können als Wirkstoffe in Arzneimitteln verwendet werden.

EP 0 240 680 A1

BAYER AKTIENGESELLSCHAFT 5090 Leverkusen, Bayerwerk
Konzernverwaltung RP
Patentabteilung Mn/ABc

## Diarylsulfidderivate

Die Erfindung betrifft neue Diarylsulfidderivate, Verfahren zu ihrer Herstellung und ihre Verwendung in Arzneimitteln.

Es wurden Diarylsulfidderivate der allgemeinen Formel (I)

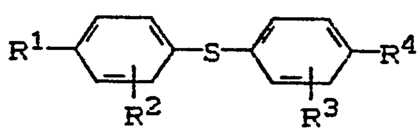
(I),

in welcher

$R^1$ - für einen Thiazolamino-Rest der Formeln

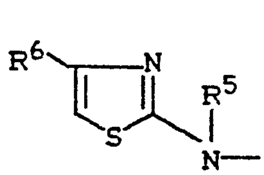
,
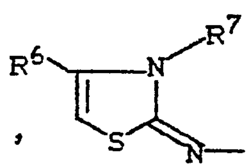
,
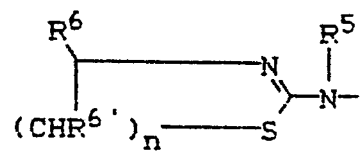

oder
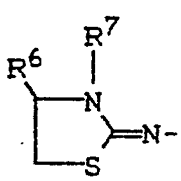
steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

$R^2$, $R^3$ - gleich oder verschieden sind und

- für Wasserstoff, Alkyl, Alkenyl, Cycloalkyl, Alkylthio, Alkoxy, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

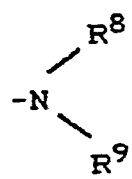

stehen,

worin

$R^8$, $R^9$ - gleich oder verschieden sind und

- für Wasserstoff, Alkyl, Aryl, Aralkyl, Acyl, Trifluoracetyl, Alkylsulfonyl, Arylsulfonyl, Trifluormethylphenylsulfonyl oder Tolylsulfonyl stehen,

und

$R^4$ - eine der oben genannten Bedeutungen von $R^1$ hat,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxylalkyl, Alkoxycarbonylalkyl, Nitro, Cyano, Halogen oder für eine Gruppe der Formel

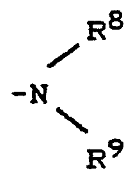

steht,

wobei
$R^8$, $R^9$ die oben angegebene Bedeutung haben,

und deren Salze, gefunden.

<u>Alkyl</u> steht hierbei im allgemeinen für einen geradkettigen oder verzweigten Kohlenwasserstoffrest mit 1 bis 12 Kohlenstoffatomen. Bevorzugt wird Niedrigalkyl mit 1 bis etwa 8 Kohlenstoffatomen. Genannt seien beispielsweise Methyl, Ethyl, Propyl, Isopropyl, Butyl, Isobutyl, Pentyl, Isopentyl, Hexyl, Isohexyl, Heptyl, Isoheptyl, Octyl und Isooctyl.

<u>Alkenyl</u> steht im allgemeinen für einen geradkettigen oder verzweigten Kohlenwasserstoffrest mit 2 bis 12 Kohlenstoffatomen und einer oder mehreren, bevorzugt mit einer oder zwei Doppelbindungen. Bevorzugt ist der Niederalkenylrest mit 2 bis etwa 6 Kohlenstoffatomen und einer Doppelbindung. Besonders bevorzugt ist ein Alkenylrest mit 2 bis 4 Kohlenstoffatomen und einer Doppelbindung. Beispielsweise seien Vinyl, Allyl, Propenyl, Isopropenyl, Butenyl, Isobutenyl, Pentenyl, Isopentenyl, Hexyl, Isohexenyl, Heptenyl, Isoheptenyl, Octenyl, Isooctenyl genannt.

<u>Cycloalkyl</u> steht im allgemeinen für einen cyclischen Kohlenwasserstoffrest mit 5 bis 8 Kohlenstoffatomen. Bevorzugt ist der Cyclopentyl- und der Cyclohexylrest. Beispielhaft seien Cyclopentyl, Cyclohexyl und Cycloheptyl genannt.

<u>Alkoxy</u> steht im allgemeinen für einen über ein Sauerstoffatom gebundenen, geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

<u>Alkylthio</u> steht im allgemeinen für einen über ein Schwefelatom gebundenen, geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio,

Isobutylthio, Pentylthio, Isopentylthio, Hexylthio, Isohexylthio, Heptylthio, Isoheptylthio, Octylthio und Isooctylthio genannt.

<u>Halogenalkyl</u> steht im allgemeinen für geradkettiges oder verzweigtes Niedrigalkyl mit 1 bis etwa 6 Kohlenstoffatomen und einem oder mehreren Halogenatomen, bevorzugt mit einem oder mehreren Fluor-, Chlor- und/oder Bromatomen. Bevorzugt ist Alkyl mit 1 bis 4 Kohlenstoffatomen und mit einem oder mehreren Fluor- und/oder Chloratomen. Besonders bevorzugt ist Alkyl mit 1 bis 2 Kohlenstoffatomen und mit bis zu fünf Fluoratomen oder mit bis zu drei Chloratomen. Beispielhaft seien genannt: Fluormethyl, Chlormethyl, Brommethyl, Fluorethyl, Chlorethyl, Bromethyl, Fluorpropyl, Chlorpropyl, Brompropyl, Fluorbutyl, Chlorbutyl, Brombutyl, Fluorisopropyl, Chlorisopropyl, Bromisopropyl, Difluormethyl, Trifluormethyl, Dichlormethyl, Trichlormethyl, Difluorethyl, Dichlorethyl, Trifluorethyl, Tetrafluorethyl, Pentafluorethyl, Trichlorethyl, Trifluorpropyl. Ganz besonders bevorzugt sind Trifluormethyl, Difluormethyl, Fluormethyl, Chlormethyl und Trifluorethyl.

<u>Halogenalkoxy</u> steht im allgemeinen für ein über ein Sauerstoffatom gebundenes geradkettiges oder verzweigtes Niedrigalkyl mit 1 bis etwa 6 Kohlenstoffatomen und einem oder mehreren Halogenatomen, bevorzugt mit einem oder meh-

reren Fluor-, Chlor- und/oder Bromatomen. Bevorzugt ist Halogenalkoxy mit 1 bis 4 Kohlenstoffatomen und mit einem oder mehreren Fluor- und/oder Chloratomen. Besonders bevorzugt ist Halogenalkoxy mit 1 bis 2 Kohlenstoffatomen und mit bis zu 5 Fluoratomen oder mit bis zu 3 Chloratomen. Beispielsweise seien genannt: Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Brombutoxy, Fluorisopropoxy, Chlorisopropoxy, Bromisopropoxy, Difluormethoxy, Trifluormethoxy, Dichlormethoxy, Trichlormethoxy, Difluorethoxy, Trifluorethoxy, Tetrafluorethoxy, Pentafluorethoxy, Trichlorethoxy, Trifluorpropoxy. Ganz besonders bevorzugt sind Trifluormethoxy, Difluormethoxy, Fluormethoxy, Chlormethoxy und Trifluorethoxy.

Halogenalkylthio steht im allgemeinen für ein über ein Schwefelatom gebundenes, geradkettiges oder verzweigtes Niedrigalkyl mit 1 bis etwa 6 Kohlenstoffatomen und einem oder mehreren Halogenatomen, bevorzugt mit einem oder mehreren Fluor-, Chlor- und/oder Bromatomen. Bevorzugt ist Halogenalkylthio mit 1 bis 4 Kohlenstoffatomen und mit einem oder mehreren Fluor- oder Chloratomen. Besonders bevorzugt ist Halogenalkylthio mit 1 bis 2 Kohlenstoffatomen und mit bis zu fünf Fluoratomen oder mit bis zu 3 Chloratomen. Beispielsweise seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Brombutylthio, Chlorbutylthio, Fluorisopropylthio, Chlorisopropylthio, Bromisopropylthio, Difluormethylthio, Trifluormethylthio, Dichlormethylthio, Trichlormethylthio, Di-

fluorethylthio, Trifluorethylthio, Tetrafluorethylthio, Pentafluorethylthio, Trichlorethylthio, Trifluorpropylthio. Ganz besonders bevorzugt sind Trifluormethylthio, Difluormethylthio, Fluormethylthio, Chlormethylthio und Trifluorethylthio.

<u>Aryl</u> steht im allgemeinen für einen aromatischen Rest mit 6 bis etwa 12 Kohlenstoffatomen. Bevorzugte Arylreste sind Phenyl, Naphthyl und Biphenyl.

<u>Aralkyl</u> steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatomen im aromatischen Teil. Beispielsweise seien folgende Aralkylreste genannt: Benzyl, Naphthylmethyl, Phenethyl und Phenylpropyl.

<u>Aryloxy</u> steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

<u>Aralkoxy</u> steht im allgemeinen für einen Aralkylrest mit 7 bis 14 Kohlenstoffatomen, wobei die Alkylkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis 14 Kohlenstoffatomen, wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Acyl steht im allgemeinen für Phenyl oder geradkettiges oder verzweigtes Niedrigalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Alkoxycarbonyl kann beispielsweise durch die Formel

-CO-Alkyl<br>
 ||<br>
 O

dargestellt werden. Alkyl steht hierbei für einen geradkettigen oder verzweigten Kohlenwasserstoffrest mit 1 bis 8 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl, Isobutoxycarbonyl.

Carboxyalkyl steht im allgemeinen für einen geradkettigen oder verzweigten Kohlenwasserstoffrest mit 1 bis 12 Kohlenstoffatomen, der durch eine Carboxygruppe substituiert ist. Bevorzugt wird Carboxyniederalkyl mit 1 bis etwa 6 Kohlenstoffatomen. Beispielsweise seien genannt: Carboxymethyl, 1-Carboxyethyl, 1-Carboxypropyl, 1-Carboxybutyl, 1-Carboxypentyl, 1-Carboxyhexyl, 2-Carboxyethyl, 2-Carboxypropyl, 2-Carboxybutyl, 3-Carboxypropyl, 3-Carboxybutyl, 4-Carboxybutyl, 2-Carboxy-1-propyl, 1-Carboxy-1-propyl.

Alkoxycarbonylalkyl steht im allgemeinen für einen geradkettigen oder verzweigten Kohlenwasserstoffrest mit 1 bis 12 Kohlenstoffatomen, der durch eine Alkoxycarbonylgruppe substituiert ist, wobei Alkoxycarbonyl die oben angegebene Bedeutung hat. Bevorzugt ist Niederalkoxycarbonylniederalkyl mit jeweils 1 bis etwa 6 Kohlenstoffatomen in jedem Alkylteil. Beispielsweise seien genannnt: Methoxycarbonylmethyl, Ethoxycarbonylmethyl, Propoxycarbonylmethyl, Butoxycarbonylmethyl, Isopropoxycarbonylmethyl, Isobutoxycarbonylmethyl, 1-Methoxycarbonyl-ethyl, 1-Ethoxycarbonyl-ethyl, 1-Propoxycarbonyl-ethyl, 1-Butoxycarbonylethyl, 1-Isopropoxycarbonyl-ethyl, 1-Isobutoxycarbonylethyl, 2-Methoxycarbonyl-ethyl, 2-Ethoxycarbonyl-ethyl, 2-Propoxycarbonyl-ethyl, 2-Butoxycarbonyl-ethyl, 2-Isopropoxycarbonyl-ethyl, 2-Isobutoxycarbonyl-ethyl, 2-Methoxycarbonyl-2-propyl, 2-Ethoxycarbonyl-2-propyl, 2-Propoxycarbonyl-2-propyl, 2-Butoxycarbonyl-2-propyl, 2-Isopropoxycarbonyl-2-propyl, 2-Isobutoxycarbonyl-2-propyl, 1-Methoxycarbonyl-2-propyl, 1-Ethoxycarbonyl-2-propyl,

1-Propoxycarbonyl-2-propyl, 1-Butoxycarbonyl-2-propyl, 1-Isopropoxycarbonyl-2-propyl, 1-Isobutoxycarbonyl-2-propyl, 3-Methoxycarbonyl-propyl, 3-Ethoxycarbonyl-propyl, 3-Propoxycarbonyl-propyl, 3-Isopropoxycarbonyl-propyl, 3-Butoxycarbonyl-propyl, 3-Isobutoxycarbonyl-propyl.

Halogen steht im allgemeinen für Fluor, Chlor, Brom oder Iod, bevorzugt für Fluor, Chlor, Brom. Besonders bevorzugt steht Halogen für Fluor oder Chlor.

Alkylsulfonyl steht im allgemeinen für einen geradkettigen, oder verzweigten Kohlenwasserstoffrest mit 1 bis 12 Kohlenstoffatomen, der über eine $SO_2$-Gruppe gebunden ist. Bevorzugt ist Niedrigalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Bevorzugt werden Verbindungen der allgemeinen Formel (I), in denen

$R^1$ - für einen Thiazolaminorest der Formel

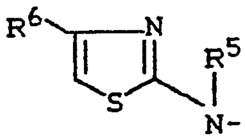 , 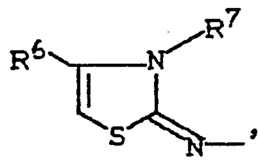 , 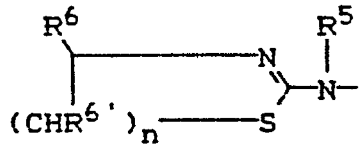

oder 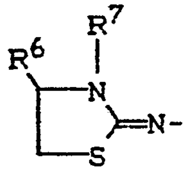 steht,

worin

$R^5$ - für Wasserstoff, Niederalkyl, Benzyl, Benzoyl oder Acetyl steht,

$R^6$, $R^{6'}$ - gleich oder verschieden sind und für Wasserstoff, Niederalkyl oder Phenyl steht,

$R^7$ - für Niederalkyl, Cyclohexyl, Benzyl, Acetyl, Benzoyl oder Phenyl steht, und

n - für eine Zahl 1 oder 2 steht,

$R^2$, $R^3$ - gleich oder verschieden sind und für Wasserstoff, Niederalkyl, Niederalkenyl, Niederalkoxy, Niederalkylthio, Halogenniederalkyl, Halogenniederalkoxy, Trifluormethylthio, Phenyl, Benzyl, Phenoxy, Benzyloxy, Niedrigalkylcarbonyl, Benzoyl, Carboxy, Niedrigalkoxycarbonyl, Carboxyniedrigalkyl, Niedrigalkoxycarbonylniedrigalkyl, Nitro, Cyano, Fluor, Chlor, Brom oder für die Gruppe der Formel

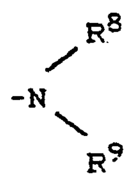 stehen,

worin
$R^8$, $R^9$ - gleich oder verschieden sind und

- für Wasserstoff, Niedrigalkyl, Phenyl, Benzyl, Niedrigalkylcarbonyl, Benzoyl, Trifluoracetyl, Niederalkylsulfonyl, Phenylsulfonyl, Trifluormethylphenylsulfonyl oder Tolylsulfonyl stehen,

und

$R^4$ - eine der oben genannten Bedeutungen von $R^1$ hat,

oder

- für Wasserstoff, Niederalkyl, Niederalkenyl, Niederalkoxy, Niederalkylthio, Halogenniederalkyl, Halogenniederalkoxy, Trifluormethylthio, Phenyl, Benzyl, Phenoxy, Benzyloxy, Niederalkylcarbonyl, Benzoyl, Carboxy, Niederalkoxycarbonyl, Carboxyniederalkyl, Niederalkoxyniederalkyl, Nitro, Cyano, Fluor, Chlor, Brom oder für eine Gruppe der Formel

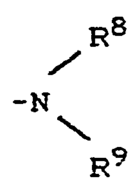

steht,

worin
$R^8$, $R^9$ die oben angegebene Bedeutung haben,

und deren Salze.

Besonders bevorzugt werden Verbindungen der Formel (I), in denen

$R^1$ - für einen Thiazolaminorest der Formel

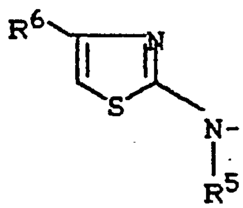
,
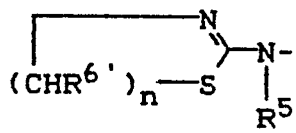
oder
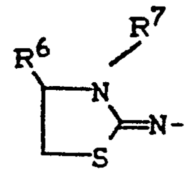

steht, worin

$R^5$ - für Wasserstoff, Methyl, Ethyl, Propyl, Isopropyl, Benzyl oder Acetyl steht,

$R^6$, $R^{6'}$ - gleich oder verschieden sind und für Wasserstoff, Methyl, Ethyl, Propyl, Isopropyl, Butyl, Isobutyl oder Phenyl steht,

und

$R^7$ - für Methyl, Ethyl oder Acetyl steht,

$R^2$, $R^3$ - gleich oder verschieden sind und
- für Wasserstoff, Methyl, Ethyl, Propyl, Isopropyl, Allyl, Methoxy, Methylthio, Trifluormethyl, Trifluormethoxy, Difluormethoxy, Trifluormethylthio, Acetyl, Carboxy, Carboxymethyl, Methoxycarbonyl, Ethoxycarbonyl, Methoxycarbonylmethyl, 1-Methoxycarbonyl-ethyl, 2-Methoxycarbonyl-ethyl, 2-Ethoxycarbonyl-methyl, 1-Ethoxycarbonyl-ethyl, 2-Ethoxycarbonyl-ethyl, Cyano, Fluor, Chlor oder Brom stehen,

$R^4$ - eine der oben genannten Bedeutungen von $R^1$ hat, oder

- für Wasserstoff, Methyl, Ethyl, Propyl, Isopropyl, Butyl, tert-Butyl, Methoxy, Methylthio, Trifluormethyl, Trifluormethoxy, Trifluormethylthio, Acetyl, Carboxy, Methoxycarbonyl, Ethoxycarbonyl, Carboxymethyl, Methoxycarbonyl-methyl, Ethoxycarbonylmethyl, 1-Methoxycarbonyl-ethyl, 2-Methoxycarbonylethyl, 1-Ethoxycarbonyl-ethyl, 2-Ethoxycarbonylethyl, Nitro, Cyano, Fluor, Chlor, Brom oder eine Gruppe der Formel

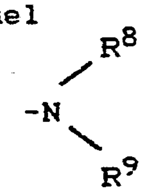

steht,

worin

$R^8$, $R^9$ - gleich oder verschieden sind und

- für Wasserstoff, Methyl, Ethyl, Propyl, Isopropyl, Phenyl, Benzyl, Acetyl, Trifluoracetyl, Methylsulfonyl, Phenylsulfonyl, Trifluormethylphenylsulfonyl oder Toluylsulfonyl stehen,

und deren Salze.

Die erfindungsgemäßen Diarylsulfidderivate können auch in Form ihrer Salze vorliegen. Im allgemeinen seien hier Salze der erfindungsgemäßen Stoffe mit organischen und anorganischen Säuren genannt.

Im Rahmen der vorliegenden Erfindung werden physiologisch unbedenkliche Salze bevorzugt. Physiologisch unbedenkliche Salze der Diarylsulfidderivate können Salze mit anorganischen oder organischen Säuren sein. Bevorzugt sind Salze mit Mineralsäuren wie beispielsweise Hydrochloride, Hydrobromide, Hydrogensulfate, Sulfate, Hydrogenphosphate, Phosphate, oder mit organischen Carbonsäuren wie Milchsäure, Maleinsäure, Fumarsäure, Essigsäure, Weinsäure, Citronensäure, Äpfelsäure oder Benzoesäure.

Beispielsweise seien die folgenden Diarylsulfidderivate genannt:

Bis-[4-($\Delta^2$-thiazolin-2-yl)aminophenyl]sulfid,
Bis-[4-(4-phenyl-$\Delta^2$-thiazolin-2-yl)aminophenyl]sulfid,
[2-Phenylthio-5-($\Delta^2$-thiazolin-2-yl)amino_7phenylessigsäure
[2-Phenylthio-5-($\Delta^2$-thiazolin-2-yl)amino]phenylessigsäuremethylester,
Bis-[4-(5-Methyl-$\Delta^2$-thiazolin-2-yl)aminophenyl]sulfid,
Bis-[4-(4-Methyl-$\Delta^2$-thiazidin-2-yl)aminophenyl]sulfid,
4-($\Delta^2$-Thiazolin-2-yl)amino-4'-aminodiphenylsulfid,
4-($\Delta^2$-Thiazolin-2-yl)amino-4'-tosylaminodiphenylsulfid,
4-(3-Methylthiazolidin-2-yl)imino-4'-tosylaminodiphenylsulfid,
4,4'-(3,3'-Diacetylthiazolidin-2-yl)diimino-diphenylsulfid,
4-(3-Methylthiazolidin-2-yl)imino-4'-amino-diphenylsulfid,
4-($\Delta^2$-Thiazolin-2-yl)amino-4'-chlor-diphenylsulfidhydrochlorid,
4-($\Delta^2$-Thiazolin-2-yl)amino-4'-methyl-diphenylsulfid,
4-Methyl-4'-(3-methylthiazolidin-2-yl)imino-diphenylsulfid,

4-($\Delta^2$-Thiazolin-2-yl)amino-4'-thiazol-2-yl-amino-diphenylsulfid,
4-Nitro-4'-($\Delta^2$-thiazol-2-yl)amino-diphenylsulfid,
4-($\Delta^2$-Thiazolin-2-yl)amino-4'-(3-trifluormethylphenyl)-sulfonylamino-diphenylsulfid,
Bis-[4-(5-Methyl-$\Delta^2$-thiazolin-2-yl)aminophenyl]sulfid.

Ein Verfahren zur Herstellung von Diarylsulfidderivaten der Formel

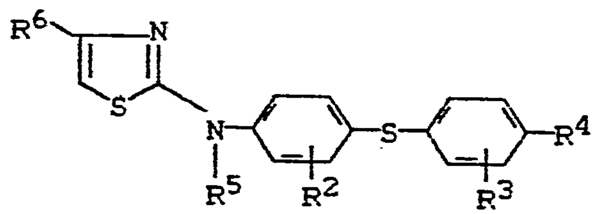

(Ia),

in welcher

$R^5$ - für Wasserstoff, Alkyl, Aralkyl oder Acyl steht und

$R^6$ - für Wasserstoff, Alkyl oder Aryl steht,

$R^2$, $R^3$ - gleich oder verschieden sind und für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

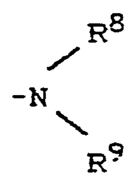

stehen,

worin

$R^8$, $R^9$ - gleich oder verschieden sind und - für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formel

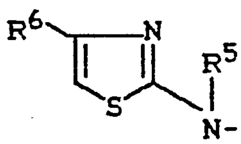 , 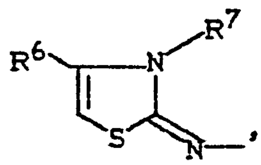 , 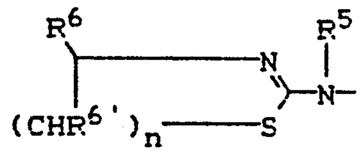

oder 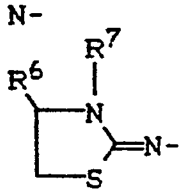 steht,

worin

$R^5$ - für Wasserstoff, Alkyl, Aralkyl oder Acyl steht,

$R^6$, $R^{6'}$ - gleich oder verschieden sind und für Wasserstoff, Alkyl , Aralkyl oder Aryl steht,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy,

Aralkylthio, Acyl, Carboxy, Alkylcarbonyl, Carboxyalkyl, Alkoxycarbonylalkyl, Nitro, Cyano, Halogen oder für eine Gruppe der Formel

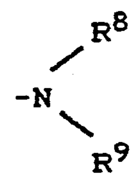

steht,

worin
$R^8$, $R^9$ - die oben angegebene Bedeutung haben,

ist dadurch gekennzeichnet, daß man

[A] Halogenverbindungen der allgemeinen Formel (II)

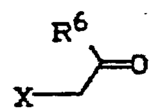

(II),

in welcher

$R^6$ - die oben angegebene Bedeutung hat und
X - für Chlor, Brom oder Iod, bevorzugt für Chlor oder Brom steht,

mit Thioharnstoffen der allgemeinen Formel (III)

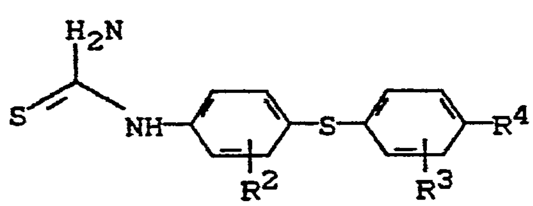

(III),

in welcher

$R^2$ - $R^4$ die oben angegebene Bedeutung haben,

gegebenenfalls in Wasser und/oder inerten organischen Lösungsmitteln, gegebenenfalls in Anwesenheit von Basen umsetzt, gegebenenfalls anschließend anfallende Salze mit Basen in freie Verbindungen überführt und diese gegebenenfalls alkyliert, aralkyliert oder acyliert,

oder daß man

[B] Thiazole der allgemeinen Formel (IV)

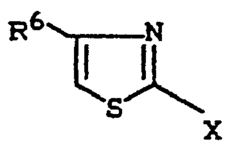

(IV),

in welcher

X - für Chlor, Brom oder Iod, insbesondere für Chlor oder Brom steht, und

$R^6$ - die oben angegebene Bedeutung hat,

mit Aminen der allgemeinen Formel (V)

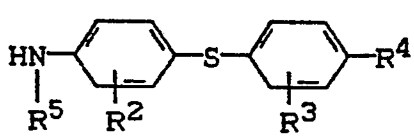

(V),

in welcher

$R^2$ - $R^5$ die oben angegebene Bedeutung haben,

in Wasser und/oder inerten organischen Lösungsmitteln, gegebenenfalls in Anwesenheit eines Katalysators umsetzt, und gegebenenfalls anfallende Salze mit Basen in die freien Verbindungen überführt,

oder daß man

[C] Thiophenole der allgemeinen Formel (VI)

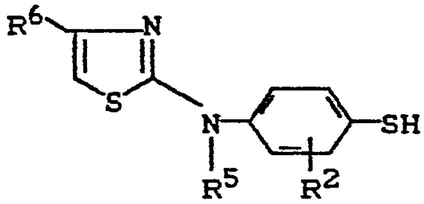

(VI),

in welcher

$R^2$, $R^5$, $R^6$ - die oben angegebene Bedeutung haben,

mit Halogenarylen der allgemeinen Formel (VII)

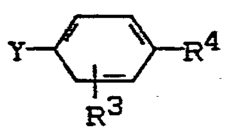

(VII),

in welcher

$R^3$, $R^4$ - die oben angegebene Bedeutung haben und

Y - für Chlor, Brom oder Iod, bevorzugt für Iod oder Brom steht,

gegebenenfalls in Wasser und/oder inerten organischen Lösungsmitteln in Anwesenheit einer Base, gegebenenfalls in Gegenwart eines Katalysators umsetzt.

Die erfindungsgemäßen Verfahren A, B und C können durch folgende Schemata veranschaulicht werden:

[A]

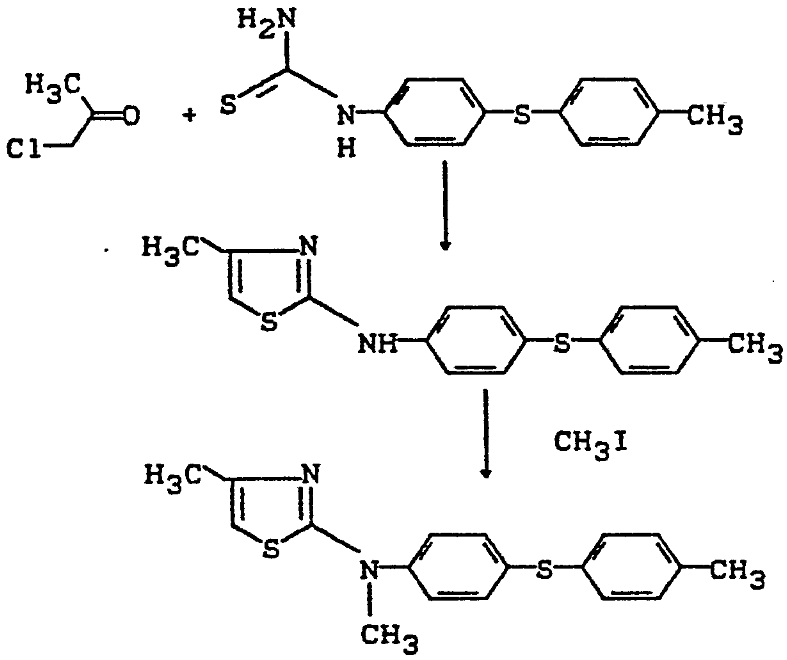


[B]

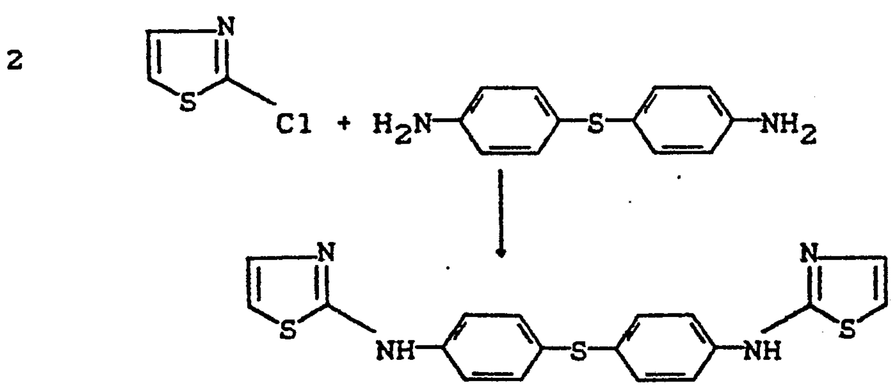

[C]

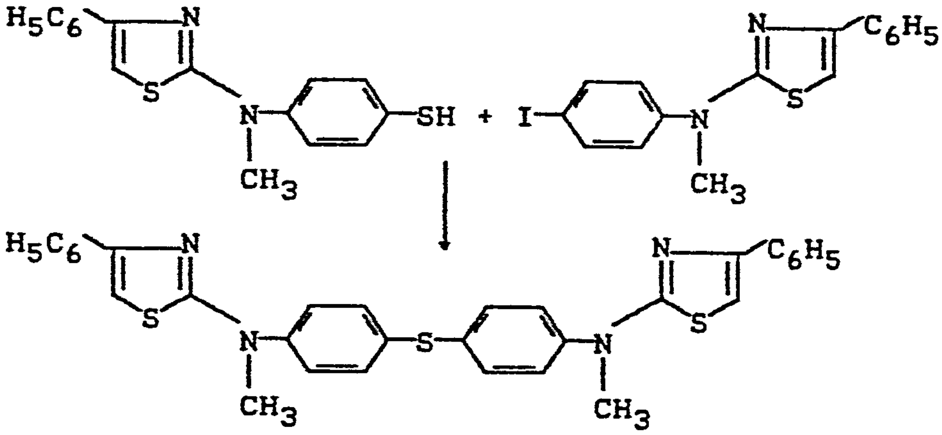

Als Lösungsmittel für die erfindungsgemäßen Verfahren A, B und C eignen sich die üblichen organischen Lösungsmittel, die sich unter den Reaktionsbedingungen nicht verändern. Hierzu gehören vorzugsweise Alkohole wie beispielsweise Methanol, Ethanol, Propanol oder Isopropanol, oder Ether wie beispielsweise Diethylether, Dioxan, Tetrahydrofuran, Glykolmono- oder -dimethylether, oder Kohlenwasserstoffe wie beispielsweise Benzol, Toluol, Xylol, Hexan, Cyclohexan, Erdölfraktionen oder Decalin, Dimethylformamid, Hexamethylphosphorsäuretriamid, Acetonitril, Essigester oder Dimethylsulfoxid. Ebenso ist es möglich, Gemische der genannten Lösungsmittel einzusetzen.

Als Basen für die erfindungsgemäßen Verfahren können die üblichen anorganischen oder organischen Basen eingesetzt werden. Hierzu gehören vorzugsweise Alkalihydroxide wie Natriumhydroxid oder Kaliumhydroxid, Alkalicarbonate wie Natriumcarbonat, Natriumhydrogencarbonat oder Kaliumcarbonat, Alkalialkoholate wie Natriummethanolat oder Kalium-tert-butanolat, oder organische Amine wie Triethylamin, Pyridin, Picolin, N-Methylpiperidin, Piperidin oder Morpholin.

Bei den Verfahrensvarianten B, C ist es bevorzugt, in Anwesenheit einer katalytischen Menge von Kupfer- oder Eisenpulver, insbesondere von Kupferpulver, zu arbeiten.

Die Herstellung der erfindungsgemäßen Verbindungen der Formel (Ia) nach Verfahren A, B, C erfolgt im allgemeinen in einem Temperaturbereich von 0° C bis 250° C, bevorzugt von 20° C bis 150° C.

Die erfindungsgemäßen Verfahren werden im allgemeinen bei Normaldruck durchgeführt. Es ist jedoch auch möglich, bei niedrigerem oder höherem Druck zu arbeiten, beispielsweise in einem Bereich von 0,5 bis 5 bar.

Bei der Durchführung des Verfahrens A wird im allgemeinen der Thioharnstoff (III) in einer Menge von 0,5 bis 5 Mol, bevorzugt von 1 bis 2 Mol bezogen auf 1 Mol der Halogenverbindung (II) eingesetzt. Das erfindungsgemäße Verfahren wird beispielsweise folgendermaßen durchgeführt: Die Halogenverbindung, der Thioharnstoff und ein geeignetes Lösungsmittel werden gemischt und gegebenenfalls erwärmt. Das anfallende Hydrohalogenid wird mit Basen in üblicher Weise in die freie Verbindung überführt und diese dann gegebenenfalls mit Verbindungen der allgemeinen Formel (VIII),

$$R^{5'} - Z \qquad \text{(VIII)},$$

in welcher

$R^{5'}$ - für Alkyl, Aralkyl oder Acyl steht,

und

Z - für Halogen, bevorzugt für Chlor, Brom oder Iod steht,

gegebenenfalls in Anwesenheit einer Base wie beispielsweise Alkalihydroxide, -alkoholate oder organischen Aminen z.B. Triethylamin in geeigneten Lösungsmitteln wie Alkoholen, Dimethylformamid oder Dimethylsulfoxid umgesetzt.

Bei der Durchführung des erfindungsgemäßen Verfahrens B wird im allgemeinen das Amin der Formel V in einer Menge von 0,1 bis 5 Mol, bevorzugt von 0,5 bis 3 Mol bezogen auf 1 Mol des Thiazols IV, eingesetzt. Gegebenenfalls kann man unter Zusatz einer weiteren Base in einer Menge von 1 bis 10 Mol, bevorzugt von 1 bis 5 Mol bezogen auf 1 Mol des eingesetzten Thiazols, arbeiten. Das erfindungsgemäße Verfahren wird beispielsweise durchgeführt, indem man Amin V, Thiazol IV und gegebenenfalls Base und Katalysator mit einem geeigneten Lösungsmittel mischt und gegebenenfalls erhitzt oder gegebenenfalls unter Druck erhitzt (z.B. in einem geschlossenen Bombenrohr).

Bei der Durchführung des erfindungsgemäßen Verfahrens C wird im allgemeinen das Thiophenol (VI) in einer Menge von 1 bis 3 Mol, bevorzugt von 1 bis 1,5 Mol bezogen auf 1 Mol des Halogenaryls (VII), eingesetzt. Die Base wird im allgemeinen in einer Menge von 1 bis 5 Mol, bevorzugt von 1 bis 3 Mol bezogen auf ein Mol Thiophenol, eingesetzt.

Das Verfahren kann beispielsweise durchgeführt werden, indem man aus Thiophenol und Base in einem inerten Lösungsmittel das Thiophenolat herstellt und dieses gegebenenfalls in geeigneten Lösungsmitteln in Gegenwart von katalytischen

Mengen Kupferpulver mit Halogenarylen umsetzt. Es ist hierbei auch möglich, das Thiophenolat zu isolieren.

Die als Ausgangsstoffe verwendeten Halogenverbindungen der Formel (II) sind bekannt oder können nach bekannten Verfahren hergestellt werden (Beilstein's Handbuch der organischen Chemie 7, 283; 1 653; (1), 151).

Die als Ausgangsstoffe verwendeten Thioharnstoffe der Formel (III) sind bekannt oder können nach bekannten Methoden hergestellt werden (Houben-Weyl's "Methoden der organischen Chemie" IX, 890; IX, 887; Ullmanns Encyclopädie der Technischen Chemie XIV, 687; B. Loev et al., J. Med. Chem. 15, 1024 (1972)).

Die als Ausgangsstoffe eingesetzten Thiazole der Formel (IV) sind bekannt oder können nach bekannten Methoden hergestellt werden (Chemistry of Heterocyclic Compounds 34 / 1; P. Schatzmann, Liebigs Ann. Chem. 261, 10 (1891)).

Die als Ausgangsverbindungen eingesetzten Amine der allgemeinen Formel (V) sind bekannt oder können nach bekannten Methoden, z.B. durch Reduktion der entsprechenden Nitroverbindungen, hergestellt werden (Houben-Weyl's "Methoden der organischen Chemie" XI/1, 363).

Die als Ausgangsverbindungen eingesetzten Thiophenole der allgemeinen Formel (VI) sind bekannt oder können nach bekannten Methoden hergestellt werden (Houben-Weyl's "Methoden der organischen Chemie" IX, 12, 26, 818; X/4, 135).

Die als Ausgangsstoffe eingesetzten Halogenaryle der Formel (VII) sind bekannt oder können nach bekannten Methoden hergestellt werden (Houben-Weyl's "Methoden der organischen Chemie" V/3, 503, V/4, 517).

Ein Verfahren zur Herstellung von Diarylsulfidderivaten der Formel

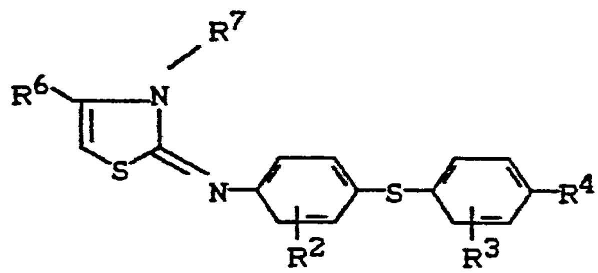

(Ib)

in welcher

$R^6$ - für Wasserstoff, Alkyl, Aralkyl oder Aryl steht,

$R^7$ - für Alkyl, Cycloalkyl, Aralkyl, Aryl oder Acyl steht,

$R^2$, $R^3$ - gleich oder verschieden sind und

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

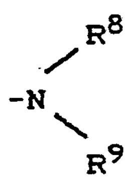

stehen,

worin

$R^8$, $R^9$ - gleich oder verschieden sind und

- für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formel

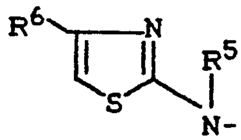

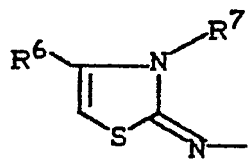

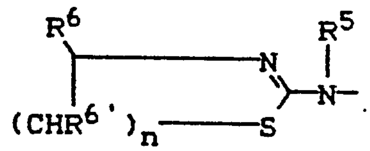

oder 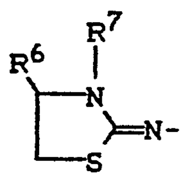 steht,

worin

$R^5$ - für Wasserstoff, Alkyl, Aralkyl oder Acyl steht,

$R^6$, $R^{6'}$ - gleich oder verschieden sind und für Wasserstoff, Alkyl oder Acyl steht,

$R^7$ für Alkyl, Cycloalkyl, Aralkyl, Aryl oder Acyl steht, und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

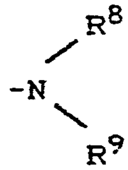

steht,

worin

$R^8$, $R^9$ - die oben angegebene Bedeutung haben,

ist dadurch gekennzeichnet, daß man

[D] Halogenverbindungen der allgemeinen Formel (II) mit substituierten Thioharnstoffen der allgemeinen Formel (IX)

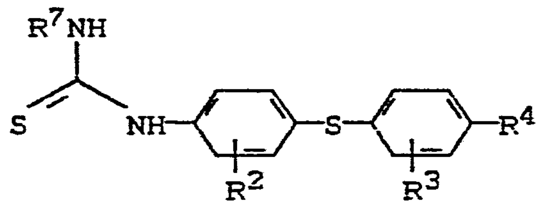

(IX),

in welcher

$R^2$-$R^4$ und $R^7$ die oben angegebene Bedeutung haben,

gegebenenfalls in Wasser und/oder inerten organischen Lösungsmitteln, gegebenenfalls in Anwesenheit einer Base umsetzt, und gegebenenfalls anfallende Salze mit Basen in die freie Verbindung überführt.

Die Herstellung der Verbindungen Ib läßt sich durch folgendes Formelschema verdeutlichen:

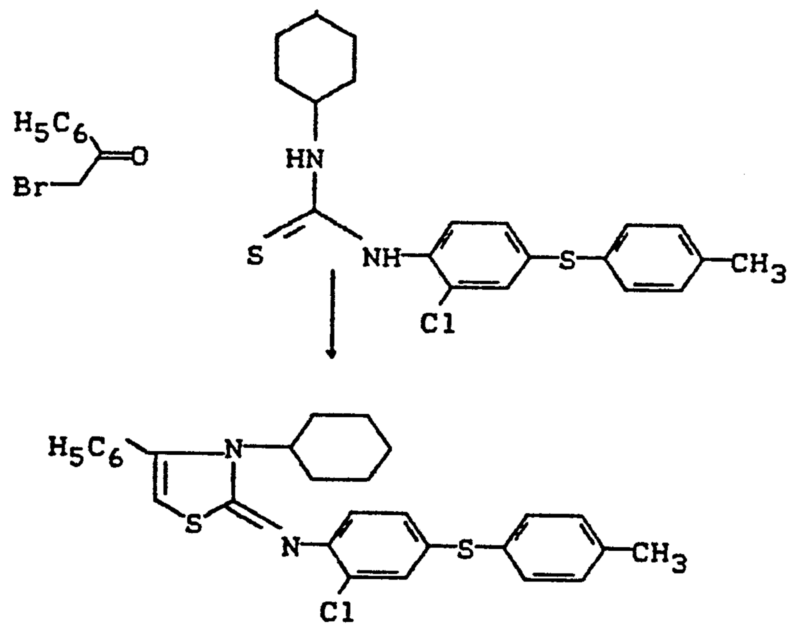

Als Lösungsmittel eignen sich Wasser oder die üblichen inerten organischen Lösungsmittel, die sich unter den Reaktionsbedingungen nicht verändern. Hierzu gehören bevorzugt Alkohole wie Methanol, Ethanol, Propanol, Isopropanol, oder Ether, wie Diethylether, Dioxan, Tetrahydrofuran, Glykolmono- oder -dimethylether, Kohlenwasserstoffe wie Benzol, Xylol, Toluol, Erdölfraktionen, oder Dimethylformamid, Dimethylsulfoxid, Hexamethylphosphorsäuretriamid, Acetonitril oder Essigester. Ebenso ist es möglich, Gemische der genannten Lösungsmittel einzusetzen.

Die Umsetzung erfolgt im allgemeinen in einem Temperaturbereich von 20°C bis 200°C, bevorzugt von 20°C bis 150°C.

Die Umsetzung wird im allgemeinen bei Normaldruck durchgeführt. Es ist aber ebenso möglich, bei erhöhtem oder erniedrigtem Druck (z.B. 0,5 - 5 bar) zu arbeiten.

Bei der Durchführung des erfindungsgemäßen Verfahrens wird im allgemeinen der substituierte Thioharnstoff IX in einer Menge von 0,1 bis 5, bevorzugt von 0,5 bis 2 mol bezogen auf 1 Mol der Halogenverbindung II eingesetzt. Das erfindungsgemäße Verfahren wird beispielsweise folgendermaßen durchgeführt: Die Halogenverbindung und der substituierte Thioharnstoff werden gemischt, gegebenenfalls in einem geeigneten Lösungsmittel gelöst und gegebenenfalls erwärmt. Das anfallende Hydrohalogenid wird mit Base in üblicher Weise in die freien Verbindungen überführt. Als Basen eignen sich hierbei die üblichen anorganischen oder organischen Basen. Hierzu gehören bevorzugt Alkalihydroxide wie Natrium- oder Kaliumhydroxid, Alkalicarbonate wie Natrium- oder Kaliumcarbonat, Natriumhydrogencarbonat, Alkalialkoholate wie Natriummethanolat oder -ethanolat oder Kaliummethanolat- oder -ethanolat, oder Triethylamin.

Die als Ausgangsverbindungen eingesetzten substituierten Thioharnstoffe sind bekannt oder können nach bekannten Methoden hergestellt werden (Houben-Weyl's "Methoden der organischen Chemie" IX, 890; Org. Synth., Coll. Vol. III, 617 (1955)).

Ein Verfahren zur Herstellung von Diarylsulfidderivaten der Formel

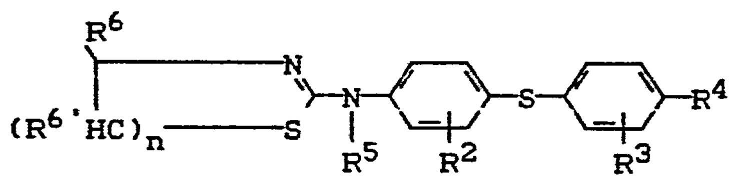

(Ic),

in welcher

$R^5$ - für Wasserstoff, Alkyl oder Acyl steht,

$R^6$, $R^{6'}$ - gleich oder verschieden sind und für Wasserstoff, Alkyl oder Aryl stehen,

und

n - für eine Zahl 1 oder 2 steht,

$R^2$, $R^3$ - gleich oder verschieden sind und

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

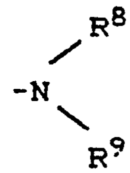

stehen,

worin

$R^8$, $R^9$ - gleich oder verschieden sind und

- für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formel

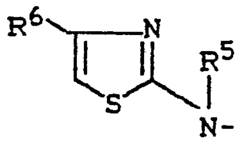

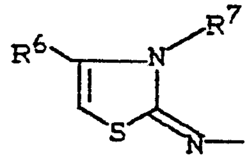

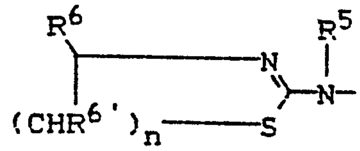

0240680

oder 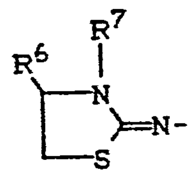 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl stehen,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

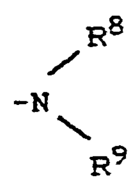 steht,

wobei

$R^8$, $R^9$ die bereits angegebene Bedeutung haben,

ist dadurch gekennzeichnet, daß man

[E] Aminoethanole der allgemeinen Formel (X)

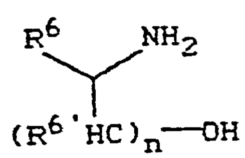

(X)

in welcher
$R^6$, $R^{6'}$ und n die oben angegebene Bedeutung haben mit Senfölen der allgemeinen Formel (XI)

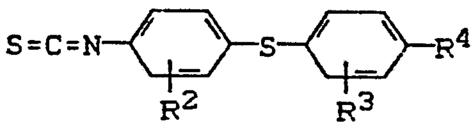
(XI)

in welcher

$R^2$ - $R^4$ die oben angegebene Bedeutung haben,

in inerten organischen Lösungsmitteln umsetzt, gegebenenfalls aryliert, alkyliert oder acyliert, oder daß man

[F] Halogensenföle der allgemeinen Formel XII

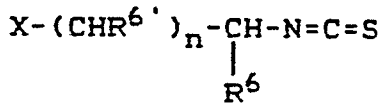
(XII)

in welcher

$R^6$, $R^{6'}$ und n die oben angegebene Bedeutung haben und

X für Chlor, Brom oder Iod, bevorzugt für Chlor oder Brom, steht,

mit Aminen der allgemeinen Formel (V),

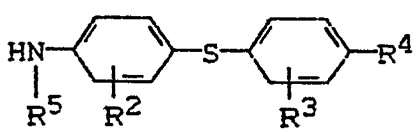
(V),

0240680

in welcher

$R^2$ bis $R^5$ die oben angegebene Bedeutung haben,

gegebenenfalls in Anwesenheit von Basen in inerten organischen Lösungsmitteln umsetzt,

oder daß man

[G] Thiophenole der allgemeinen Formel XIII

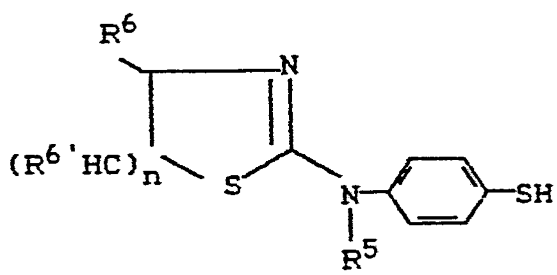

(XIII),

in welcher

$R^2$, $R^5$, $R^6$, $R^{6'}$ und n die oben angegebene Bedeutung haben,

mit Halogenarylen der allgemeinen Formel (VII), gegebenenfalls in Wasser und/oder inerten organischen Lösungsmitteln in Anwesenheit einer Base, gegebenenfalls in Gegenwart eines Katalysators, umsetzt.

Die erfindungsgemäßen Verfahren E, F und G lassen sich durch folgende Schemata verdeutlichen:

[E]

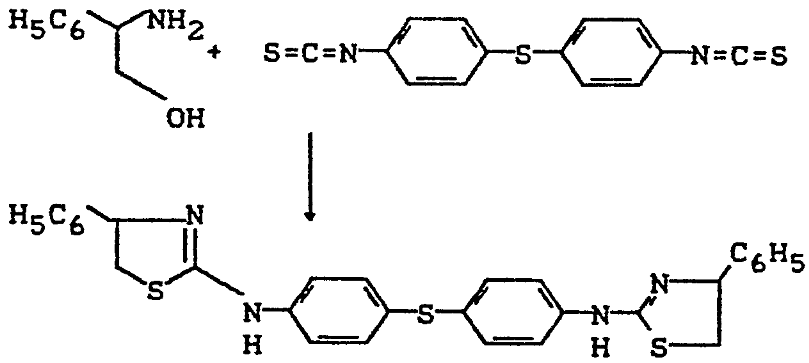

[F]

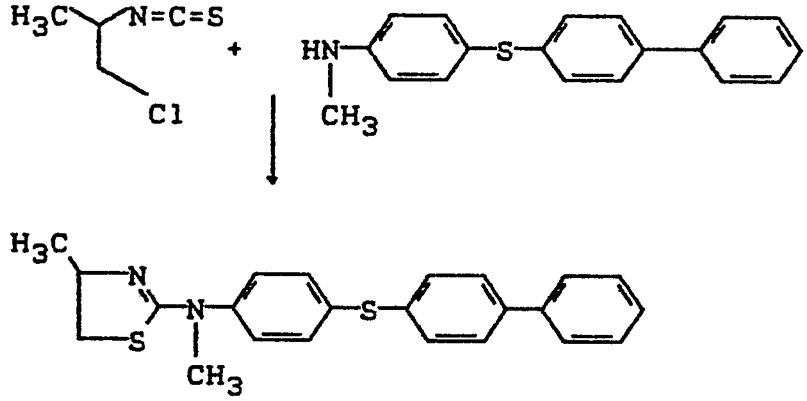

[G]

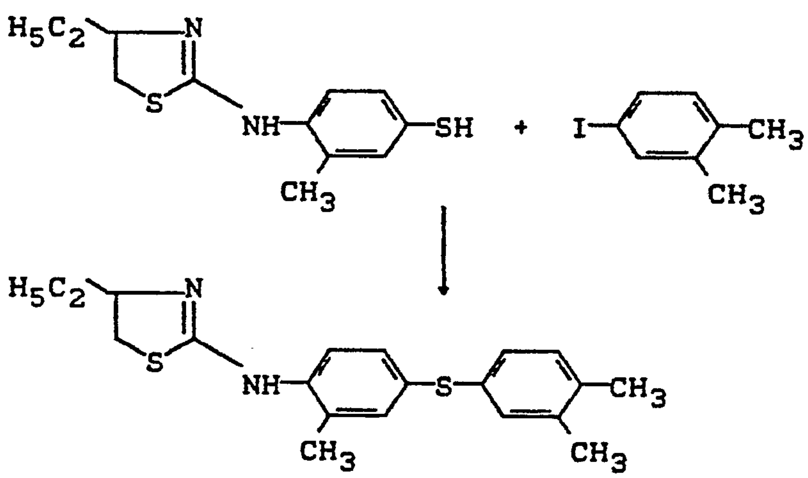

Als Lösungsmittel für die erfindungsgemäßen Verfahren E und F eignen sich die üblichen organischen Lösungsmittel, die sich unter den Reaktionsbedingungen nicht verändern.

Hierzu gehören bevorzugt Ether wie Diethylether, Dioxan, Tetrahydrofuran, Glykolmono- oder -dimethylether, oder Halogenkohlenwasserstoffe wie Methylenchlorid, Chloroform oder Tetrachlorkohlenstoff, oder Kohlenwasserstoffe wie Benzol, Toluol, Xylol, Hexan, Cyclohexan oder Erdölfraktionen. Ebenso ist es möglich, Gemische der genannten Lösungsmittel einzusetzen.

Als Basen bei Verfahren F eignen sich die üblichen anorganischen oder organischen Basen. Hierzu gehören bevorzugt Trialkylamine wie Triethylamin, oder Pyridin, Picolin, Morpholin, N-Methylpiperidin, oder Alkalihydroxide wie Natrium- oder Kaliumhydroxid, oder Alkalicarbonate wie Natrium- oder Kaliumcarbonat, oder Natriumhydrogencarbonat.

Die Umsetzungen in Verfahren E und F werden im allgemeinen in einem Temperaturbereich von $-10^{0}$C bis $+150^{0}$C, bevorzugt von $0^{0}$C bis $+80^{0}$C, durchgeführt.

Die erfindungsgemäßen Verfahren E und F werden im allgemeinen bei Normaldruck durchgeführt. Es ist jedoch auch möglich, bei erhöhtem oder erniedrigtem Druck zu arbeiten (z.B. von 0,5 bis 5 bar).

Bei der Durchführung des Verfahrens E wird im allgemeinen das Aminoethanol (X) in einer Menge von 1 bis 10, bevorzugt von 1 bis 5 Mol, bezogen auf 1 Mol Senföl (XI), eingesetzt, bei Verfahren F wird im allgemeinen 0,5 bis 10, bevorzugt 1 bis 5 Mol Halogensenföl XII, sowie 0,5 bis 10 Mol, bevorzugt 1 bis 5 Mol Base, bezogen auf 1 Mol Amin (V), eingesetzt.

Die Verfahren E und F werden beispielsweise derart durchgeführt, daß man die Komponenten mit einem geeigneten Lösungsmittel mischt und gegebenenfalls erhitzt. Die Aufarbeitung erfolgt in allgemein bekannter Weise und ist dem Fachmann vertraut.

Die Durchführung, sowie die Bedingungen für Verfahren G sind analog denen, wie sie bereits für Verfahren C beschrieben wurden.

Die als Ausgangsstoffe eingesetzten Aminoalkohole der Formel X sind bekannt oder können nach bekannten Methoden hergestellt werden (Beilstein's Handbuch der organischen Chemie XII, 182; IV, 275).

Die als Ausgangsstoffe eingesetzten Senföle der Formel XI sind bekannt oder können nach bekannten Methoden hergestellt werden (Houben-Weyl's "Methoden der organischen Chemie" IX, 875).

Die als Ausgangsstoffe eingesetzten Halogensenföle XII sind bekannt oder können nach bekannten Methoden hergestllt werden (R.E. Hackler, T.W. Balko, Synth. Commun. 5, 43 (1975)).

Die als Ausgangsstoffe eingesetzten Thiophenole der Formel XIII sind bekannt oder können nach bekannten Methoden hergestellt werden (z.B. Houben-Weyl's "Methoden der organischen Chemie, IX, 12, 16, 818; X/4, 135).

Ein Verfahren zur Herstellung von Diarylsulfidderivaten der Formel

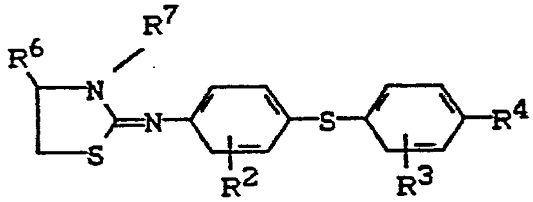

(Id)

in welcher

$R^6$ - für Wasserstoff, Alkyl oder Aryl steht, und
$R^7$ - für Alkyl, Cycloalkyl, Aralkyl, Aryl oder Acyl steht,

$R^2$, $R^3$ - gleich oder verschieden sind und
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

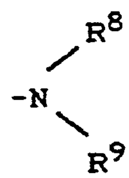

stehen,

worin
$R^8$, $R^9$ - gleich oder verschieden sind und
- für Wasserstoff, Alkyl, Aryl, Aralkyl, Acyl, Trifluoracetyl, Alkylsulfonyl, Phenylsulfonyl,

Trifluormethylphenylsulfonyl oder Tolylsulfonyl stehen,

und

$R^4$ - für einen Rest der Formel

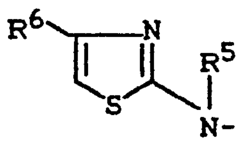
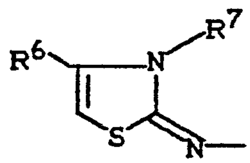
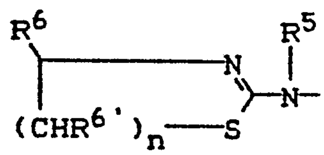

oder 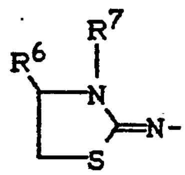 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

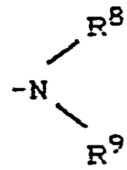

steht,

wobei

$R^8$, $R^9$ die bereits angegebene Bedeutung haben,

ist dadurch gekennzeichnet,daß man

[H] Dihalogenverbindungen der allgemeinen Formel (XIV)

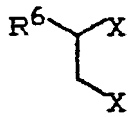

(XIV)

in welcher

$R^6$ - die angegebene Bedeutung hat und

X - für Chlor, Brom oder Iod, bevorzugt für Chlor oder Brom, steht,

mit substituierten Thioharnstoffen der allgemeinen Formel (IX)

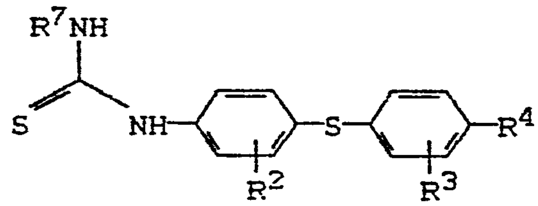

in welcher

$R^2$ bis $R^7$ die oben genannte Bedeutung haben,

gegebenenfalls in Wasser und/oder inerten organischen Lösungsmitteln, gegebenenfalls in Anwesenheit von Basen

0240680

umsetzt und gegebenenfalls anfallende Salze mit Basen in die freien Verbindungen überführt.

Die Herstellung der erfindungsgemäßen Verbindungen der Formel Id läßt sich durch folgendes Schema verdeutlichen:

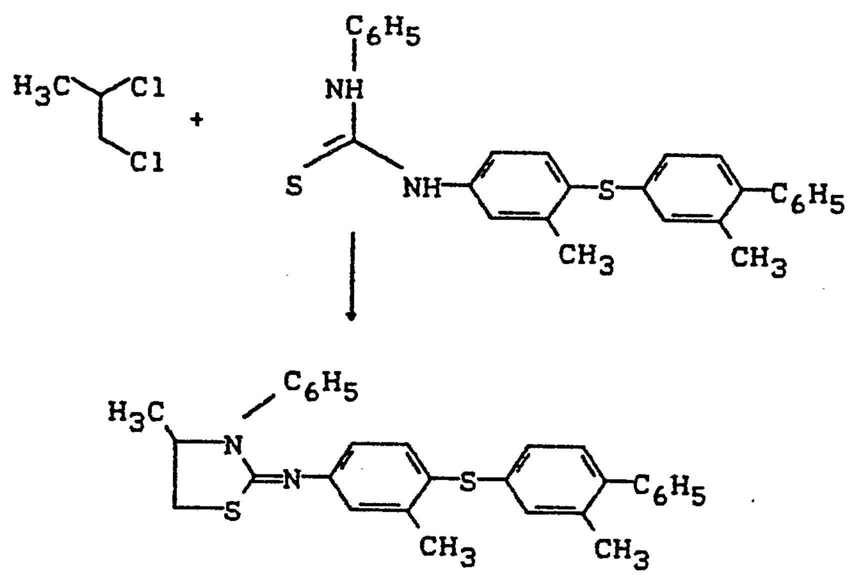

Als Lösungsmittel eignen sich Wasser oder die üblichen inerten organischen Lösungsmittel, die sich unter den Reaktionsbedingungen nicht verändern. Hierzu gehören bevorzugt Alkohole wie Methanol, Ethanol, Propanol, Isopropanol, oder Ether, wie Diethylether, Dioxan, Tetrahydrofuran, Glykolmono- oder -dimethylether, Kohlenwasserstoffe wie Benzol, Xylol, Toluol, Erdölfraktionen oder Dimethylformamid, Dimethylsulfoxid, Hexamethylphosphorsäuretriamid, Acetonitril oder Essigester. Ebenso ist es möglich, Gemische der genannten Lösungsmittel einzusetzen.

Die Umsetzung erfolgt im allgemeinen in einem Temperaturbereich von 20°C bis 200°C, bevorzugt von 20°C bis 150°C.

Die Umsetzung wird im allgemeinen bei Normaldruck durchgeführt. Es ist aber ebenso möglich, bei erhöhtem oder erniedrigtem Druck (z.B. 0,5 - 5 bar) zu arbeiten.

Bei der Durchführung des erfindungsgemäßen Verfahrens wird im allgemeinen der substituierte Thioharnstoff IX in einer Menge von 0,1 bis 5, bevorzugt von 0,5 bis 2 mol, bezogen auf 1 Mol der Halogenverbindung XIV eingesetzt. Das erfindungsgemäße Verfahren wird beispielsweise folgendermaßen durchgeführt: Die Halogenverbindung und der substituierte Thioharnstoff werden gemischt, gegebenenfalls in einem geeigneten Lösungsmittel gelöst und gegebenenfalls erwärmt. Das anfallende Hydrohalogenid wird mit Basen in üblicher Weise in die freien Verbindungen überführt. Als Basen eignen sich hierbei die üblichen anorganischen oder organischen Basen. Hierzu gehören bevorzugt Alkalihydroxide wie Natrium- oder Kaliumhydroxid, Alkalicarbonate wie Natrium- oder Kaliumcarbonat, Natriumhydrogencarbonat, Alkalialkoholate wie Natriummethanolat oder -ethanolat oder Kaliummethanolat- oder -ethanolat, oder Triethylamin.

Die als Ausgangsverbindungen eingesetzten substituierten Thioharnstoffe sind bekannt oder können nach bekannten Methoden hergestellt werden (Houben-Weyl's "Methoden der organischen Chemie" IX, 890; Org. Synth., Coll. Vol. III, 617 (1955)).

Die erfindungsgemäßen Diarylsulfide der Formel (I) können als Wirkstoffe in Arzneimitteln für die Human- und/oder Veterinärmedizin eingesetzt werden. Die Stoffe wirken überraschenderweise als Hemmer (Stimulatoren) von enzymatischen Reaktionen im Rahmen des Arachidonsäurestoffwechsels, insbesondere der Lipoxygenase.

Sie sind damit bevorzugt zur Behandlung und Verhütung von Erkrankungen der Atemwege wie Allergien, Asthma, Bronchitis, Emphysemen, Schocklunge, Herz-Kreislauf-Erkrankungen, Entzündungen, Rheuma, Psoriasis, Ödemen, Thrombosen, Thromboembolien, Ischämien (periphere, cardiale, cerebrale Durchblutungsstörungen), bei Gewebstransplantationen sowie zur Cytoprotection im Gastro-intestinaltrakt geeignet.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B.

Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Alkylsulfonate und Arylsulfonate), Dispergiermittel, Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art der Applikation, aber auch dem individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß.. Im Falle der Applikation größerer Mengen kann es empfehlenswert sein, diese in mehrere Einzelgaben über den Tag zu verteilen.

Herstellungsbeispiele

Beispiel 1

Bis(4-isothiocyanatophenyl)sulfid

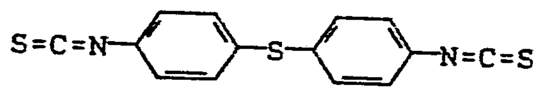

Zu einer Lösung aus 31 ml Thiophosgen in 300 ml Toluol wird langsam unter Eiskühlung eine filtrierte Lösung von 47,5 g Bis(4-aminophenyl)sulfid in 1,5 l Toluol zugetropft. Es wird 30 min. bei 0° C gerührt, auf 10° C erwärmt und eine Lösung von 110 ml Triethylamin in 150 ml Toluol zugetropft. Es wird weitere 30 min. bei 15° C und 2 h bei Raumtemperatur nachgerührt, danach wird der Niederschlag abgesaugt und die Lösung im Vakuum bei 40° C eingeengt. Der Rückstand wird an Kieselgel mit Cyclohexan chromatographiert.

Fp.: 78-79° C

Ausbeute: 64 % der Theorie

Beispiel 2

Bis[4-($\Delta^2$-thiazolin-2-yl)aminophenyl]sulfid

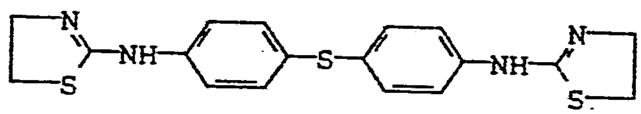

Zu einer Mischung aus 30,4 g Bis(4-isothiocyanatophenyl)-sulfid und 250 ml Ethanol werden langsam bei 0° C - 5° C 12,2 ml Aminoethanol zugetropft und 2 h bei 0° C gerührt.

0240680

Der Niederschlag wird abgesaugt. Zur Reinigung wird der Rückstand mit konzentrierter Salzsäure in der Siedehitze in Lösung gebracht und über Nacht bei Raumtemperatur gerührt. Unter Eiskühlung wird mit 45 %iger Natronlauge bis pH 9 versetzt, der Niederschlag abgesaugt, mit Wasser gewaschen und getrocknet.
Schmelzpunkt: 195-195,5° C
Ausbeute: 81 % der Theorie.

Beispiel 3

Bis[4-($\Delta^2$-thiazolin-2-yl)aminophenyl]sulfid HCl-Salz

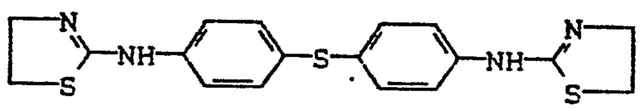 · 2 HCl

3 g Bis[4-($\Delta^2$-thiazolin-2-yl)aminophenyl]sulfid werden in 0,6 ml 1 N HCl und 10 ml $H_2O$ aufgenommen und lyophylisiert.
Ausbeute: quantitativ
Hygroskopische Kristalle zerfließen an der Luft

Beispiel 4

Bis[4-(4-phenyl-$\Delta^2$-thiazolin-2-yl)aminophenyl]sulfid

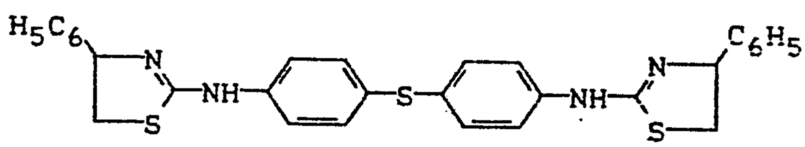

5,6 g Bis(4-isothiocyanatophenyl)sulfid und 5,5 g 2-Amino-2-phenylethanol werden in 100 ml Methylenchlorid gelöst und 1 h bei 0° C gerührt. Der Niederschlag wird

abgesaugt und in konz. HCl in der Siedehitze gelöst. Nach dem Abkühlen wird bis pH 7 mit Natronlauge versetzt. Der Niederschlag wird abgesaugt, mit Ether/Hexan = 1:1 ausgekocht und an Kieselgel chromatographiert (Laufmittel Dichlormethan).

Schmelzpunkt: 70°C Zers.

Ausbeute: 68 % d. Th.

Beispiel 5

(5-Nitro-2-phenylthio)phenylessigsäure

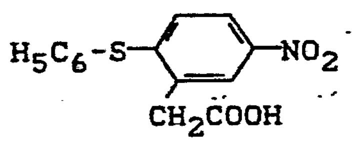

Zu einer Lösung aus 39,5 g KOH in 400 ml $H_2O$ werden 40,5 g Thiophenol eingetropft. Danach wird die Mischung portionsweise mit 71 g (2-Chlor-5-nitro)phenylessigsäure und 4 g Kupferpulver versetzt und 7 h am Rückfluß erhitzt. Die Mischung wird noch heiß filtriert, abgekühlt und mit Salzsäure angesäuert. Die wäßrige Phase wird von ausgeschiedenen Öl abdekantiert und mit Ethanol/Wasser kristallisiert.

Ausbeute: 53 % der Theorie

Schmelzpunkt: 135°C

Beispiel 6

(5-Nitro-2-phenylthio)phenylessigsäuremethylester

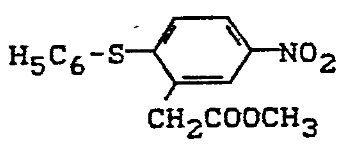

40 g (5-Nitro-2-phenylthio)phenylessigsäure in 200 ml Methanol werden mit 3 g Schwefelsäure versetzt, 2 h zum Rückfluß erhitzt, abgekühlt und die Lösung eingedampft. Der Rückstand wird mit 200 ml Ether und 200 ml Eiswasser versetzt, die Phasen getrennt und die wäßrige Phase 3 mal mit Ether extrahiert. Die vereinigten Etherphasen werden 2 mal mit gesättigter Natriumcarbonatlösung gewaschen, über Natriumsulfat getrocknet und eingeengt.
Ausbeute: 98 % der Theorie.
Schmelzpunkt: gelbes Öl, wurde nicht destilliert

Beispiel 7

(5-Amino-2-phenylthio)phenylessigsäuremethylester

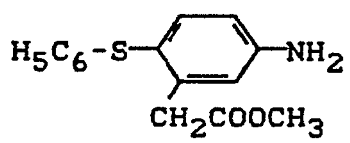

In eine Lösung von 40,3 g (5-Nitro-2-phenylthio)phenylessigsäuremethylester und 450 ml Essigsäure werden portionsweise 135,5 g Zinn-(II)-Chlorid-Hydrat eingetragen, 175 ml konz. Salzsäure zugegeben und 5 h zum Rückfluß erhitzt. Nach Abkühlen wird eingeengt, mit 550 ml 10 %iger Natronlauge verrührt, abgesaugt und wieder in 1 l verdünnter Salzsäure gelöst. Nach Stehen über Nacht erhält man 22,5 g farblose Kristalle.
Ausbeute: 63 % der Theorie.
Schmelzpunkt: 95°C Zers.

Beispiel 8

(5-Isothiocyanato-2-phenylthio)phenylessigsäuremethylester

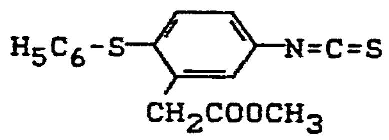

Zu einer Lösung von 9,2 g Thiophosgen in 200 ml Toluol werden 22 g (5-Amino-2-phenylthio)phenylessigsäuremethylester in 500 ml Toluol bei $0^{\circ}$ C gegeben und 4 h im Eisbad gerührt. Nach Einengen wird der Rückstand an Kieselgel mit Methylenchlorid chromatographiert.
Ausbeute: 32 % der Theorie.
Fp.: 81-$82^{\circ}$ C.

Beispiel 9

[2-Phenylthio-5-($\Delta^2$-thiazolin-2-yl)amino]phenylessigsäure

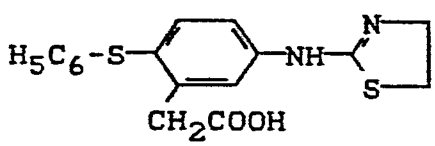

Zu 8 g (5-Isothiocyanato-2-phenylthio)phenylessigsäuremethylester in 150 ml Methylenchlorid werden bei $0^{\circ}$ C 1,8 g Aminoethanol getropft. Nach 30 min. Rühren wird eingedampft und der Niederschlag in konz. Salzsäure aufgeschlämmt und 1 h zum Sieden erhitzt. Dabei bildet sich eine klare Lösung, aus der beim Abkühlen 6,9 g Säure ausfallen.
Ausbeute: 79 % der Theorie
Schmelzpunkt: 159-$60^{\circ}$ C

Beispiel 10

[2-Phenylthio-5-($\Delta^2$-thiazolin-2-yl)amino]phenylessigsäuremethylester

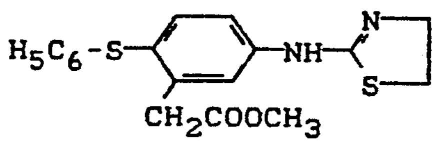

Analog Beispiel 5 erhält man 5,3 g Produkt aus [2-Phenylthio-5-($\Delta^2$-thiazolin-2-yl)amino]phenylessigsäure und Methanol.
Ausbeute: 74 % der Theorie
Schmelzpunkt: 122-4° C

Beispiel 11

4-(Phenylthio)phenylisothiocyanat

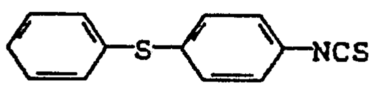

Zu einer Lösung von 11,9 g Thiophosgen in 250 ml Toluol werden bei Eiskühlung nacheinander eine Lösung von 20,1 g 4-Phenylthioanilin in 250 ml Toluol und dann 20,2 g Triethylamin zugetropft und 1 h gerührt. Nach Erwärmen auf Raumtemperatur wird über Kieselgur abgesaugt und eingedampft. Das verbleibende Öl wird mit Hexan versetzt, abfiltriert und erneut eingedampft. Nach Chromatographie an Kieselgel mit Cyclohexan erhält man ein klares Öl.
Ausbeute: 82 % der Theorie.

0240680

Beispiel 12

2-[N-(4-Phenylthio)phenyl)]amino-$\Delta^2$-thiazolin

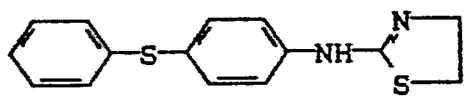

Zu einer Lösung von 20 g (4-Phenylthio)phenylisothiocyanat in 250 ml Methylenchlorid werden bei Eiskühlung 5 g Aminoethanol zugetropft und 1 h gerührt. Danach wird eine weitere Stunde im Rückfluß erhitzt, abgekühlt, abgesaugt und mit Wasser nachgewaschen. Der Rückstand wird in Methylenchlorid gelöst, die organische Phase wird mit Natronlauge gewaschen, getrocknet und eingedampft.
Ausbeute: 79 % der Theorie.
Schmelzpunkt: 99° C.

Beispiel 13

4-(4-Cyanophenylthio)phenylisothiocyanat

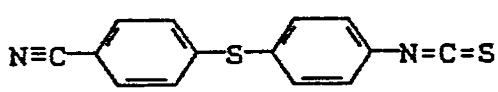

Zu einer Lösung von 2,3 g Thiophosgen in 50 ml Toluol werden bei Eiskühlung nacheinander eine Lösung von 4,3 g 4-(4-Cyanophenylthio)anilin in 100 ml Toluol und dann 3,8 g Triethylamin zugetropft, 1 h bei Eiskühlung und dann 1 h bei Raumtemperatur gerührt. Die Mischung wird über Kieselgur abgesaugt, einrotiert und der Rückstand an Kieselgel mit Cyclohexan chromatographiert.
Ausbeute: 83 % der Theorie.
Schmelzpunkt: 91° C

Beispiele 14, 15 und 16

2-[4-(4-Cyanophenylthio)phenyl]amino-$\Delta^2$-thiazolin 14

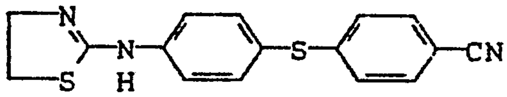

4-[4-($\Delta^2$-Thiazolin-2-yl)aminophenylthio]benzoesäure 15

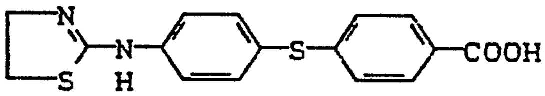

und

4-[4-($\Delta^2$-Thiazolin-2-yl)aminophenylthio]benzoesäureamid 16

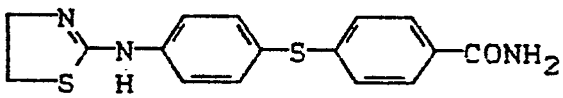

Zu einer Lösung von 4 g 4-(4-Cyanophenylthio)phenyliso- thiocyanat in 100 ml Methylenchlorid werden bei Eiskühlung 9 g Aminoethanol getropft, 1 h gerührt und abgesaugt. Der Rückstand wird in 100 ml konz. HCl gelöst, 1 h zum Rückfluß erhitzt. Nach Abkühlung wird mit NaOH bis zur alkalischen Reaktion versetzt und der entstandene Niederschlag abgesaugt.

Ausbeute: 0,1 g 14

Schmelzpunkt: 135-6° C.

Die Mutterlauge wird mit HCl angesäuert, mit Methylenchlorid versetzt und der zwischen den Phasen entstandene Niederschlag abgesaugt.

Ausbeute: 0,5 g 15

Schmelzpunkt: 210-2° C.

Die beiden Phasen werden danach getrennt und die wäßrige Phase 2 mal mit Methylenchlorid extrahiert. Die organische Phase wird über $Na_2SO_4$ getrocknet und eingedampft.
Ausbeute: 0,1 g <u>16</u>
Schmelzpunkt: 192-3°C.

Beispiel 17

Bis[4-(3-methyl-thiazolidin-2-imino)phenyl]sulfid

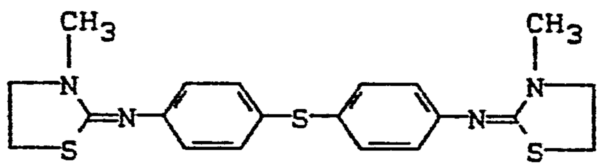

Verfahrensvariante A:

4,8 g Bis[4-($\Delta^2$-thiazolin-2-yl)aminophenyl]sulfid werden in 100 ml getrocknetem Dimethylformamid gelöst und 0,75 g Natriumhydrid zugegeben. Man läßt 2 Stunden im Ultraschallbad bei Raumtemperatur reagieren, kühlt auf 0 bis 5°C ab und gibt 1,6 ml Methyljodid zu. Man rührt 1 Stunde im Eisbad, eine weitere Stunde bei Raumtemperatur und engt dann im Vakuum zur Trockene ein. Der Rückstand wird in Methylenchlorid suspendiert und mit 2 N Salzsäure gewaschen. Die wässrige Phase wird mit 2 N Natronlauge alkalisch gemacht, im Vakuum zur Trockene eingedampft und der Rückstand im Methylenchlorid ausgerührt. Dann wird vom Unlöslichen abfiltriert, das Filtrat im Vakuum zur Trockene eingeengt und das zurückbleibende Öl über Kieselgel mit Methylenchlorid/Methanol = 9/1 chromatographiert.
Ausbeute: 4 % der Theorie
Schmelzpunkt: 130-140°C

Verfahrensvariante B:

0,9 g Bis(4-isothiocyanatophenyl)sulfid werden in 20 ml Methylenchlorid gelöst, die Lösung auf 0 bis 5°C abgekühlt und 0,2 g N-Methylethanolamin zugegeben. Man rührt 1 Stunde bei Eiskühlung und saugt ab. Der Rückstand wird in 20 ml konz. Salzsäure gelöst, 1 Stunde im Rückfluß erhitzt, abgekühlt und mit Ammoniak alkalisch gemacht. Der Niederschlag wird abgesaugt und getrocknet.
Ausbeute: 22 % der Theorie
Schmelzpunkt: 139-141°C.

Beispiel 18

4-(4-Trifluormethylphenylthio)phenylisothiocyanat

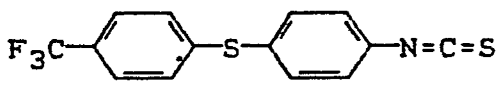

Zu einer Lösung aus 2 g Thiophosgen in 50 ml Toluol werden langsam unter Eiskühlung eine filtrierte Lösung von 4,5 g 4-(4-Trifluormethylphenyl)thio-anilin in 150 ml Toluol und anschließend 3,4 g Triethylamin zugetropft. Es wird 2 Stunden bei Eiskühlung gerührt, einrotiert und der Rückstand über eine Kieselgelsäule mit Cyclohexan chromatographiert.
Ausbeute: 77 % der Theorie
Schmelzpunkt: 46-47°C

Beispiel 19

2-[N-4-(4-Trifluormethylphenylthio)phenyl]amino-$\Delta^2$-thiazolin Hydrochlorid

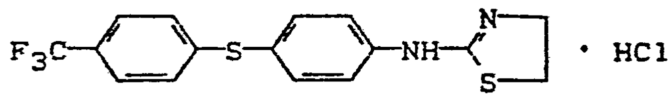

Zu einer Lösung von 4,1 g 4-(4-Trifluormethylphenylthio)phenylisothiocyanat in 50 ml Methylenchlorid werden bei Eiskühlung 0,8 g Ethanolamin eingetropft, 30 min. bei Eiskühlung gerührt und der Niederschlag abgesaugt.

Der Rückstand wird in 100 ml konzentrierter Salzsäure suspendiert, 1 Stunde im Rückfluß erhitzt und nach Abkühlen mit Ammoniak alkalisch gemacht. Der Rückstand wird abgesaugt, in Salzsäure aufgenommen und erneut mit Ammoniak alkalisch gestellt. Der Niederschlag wird abgesaugt, über Nacht in Wasser gerührt, wiederum abgesaugt und getrocknet.
Ausbeute: 74% der Theorie
Schmelzpunkt: 78-80° C

Beispiel 20

Bis[4-(3-cyclohexyl-5-methyl-thiazolidin-2-imino)phenyl] sulfid

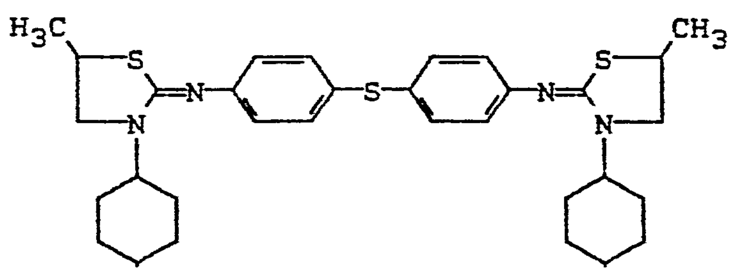

Zu einer Mischung von 10 g Bis(4-isothiocyanatophenyl)-sulfid in 100 ml Methylenchlorid wird unter Eiskühlung eine Lösung von 5,2 g N-Cyclohexylamino-1-methylethanol

in 20 ml Methylenchlorid zugetropft, 1 Stunde bei 0 bis 5° C gerührt und abgesaugt. Der Rückstand wird in 150 ml konzentrierter Salzsäure suspendiert und 1 Stunde im Rückfluß erhitzt. Nach Abkühlen wird mit Ammoniak ausgefällt, der Rückstand erneut in konz. Salzsäure aufgenommen und wieder mit Ammoniak alkalisch gestellt. Der Niederschlag wird abgesaugt und getrocknet. Der Rückstand wird über eine Kieselgelsäule mit Methylenchlorid/Methanol = 50:1 chromatographiert.
Ausbeute: 2 % der Theorie
Schmelzpunkt: 198° C

Beispiel 21

2-[4-(4-Aminophenylthio)phenyl]imino-3-phenyl-thiazolidin

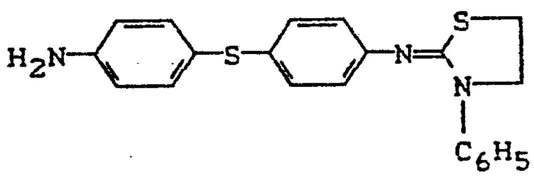

Zu einer Mischung von 10 g Bis(4-isocyanatophenyl)sulfid in 100 ml Methylenchlorid wird bei Eiskühlung 9,2 g Phenylethanolamin zugetropft und 1 Stunde bei Eiskühlung gerührt. Der Rückstand wird abgesaugt, in 100 ml konz. Salzsäure suspendiert, 1 Stunde im Rückfluß erhitzt, nach dem Abkühlen mit Ammoniak alkalisch gemacht und wieder abgesaugt. Der Rückstand wird in konz. Salzsäure gelöst, erneut mit Ammoniak alkalisch gemacht, abgesaugt und getrocknet. Der Rückstand wird über Kieselgel mit Methylenchlorid/Methanol = 50:1 chromatographiert.
Ausbeute: 6 % der Theorie
Schmelzpunkt: 143-145° C

Beispiel 22

Bis[4-(5-methyl-Δ²-thiazolin-2-yl)aminophenyl]sulfid

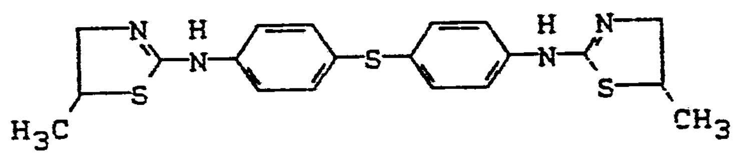

10 g (0,033 mol) Bis(4-isothiocyanatophenyl)sulfid werden in 100 ml Dichlormethan gelöst, auf 0 - 5° C abgekühlt und tropfenweise mit 5,0 g ≡ 5,2 ml (0,066 mol) Amino-2-hydroxypropan versetzt. Man rührt noch ca. 1 h bei 0 - 5° C nach, dampft im Vakuum zur Trockene ein und suspendiert den Rückstand in konz. Salzsäure. Die Suspension erhitzt man 1 h am Rückfluß, wobei sich langsam eine klare Lösung bildet. Nach dem Abkühlen fällt man das Produkt unter Rühren durch vorsichtigen Zusatz einer konzentrierten Ammoniaklösung aus; ggfs. Umfällen mit Salzsäure / Ammoniak.

Ausbeute: 1,2 g (8,7% der Theorie)

Schmp. : 192° C (Zers.)

Beispiel 23

Bis[4-(4-methyl-Δ²-thiazolin-2-yl)aminophenyl]sulfid

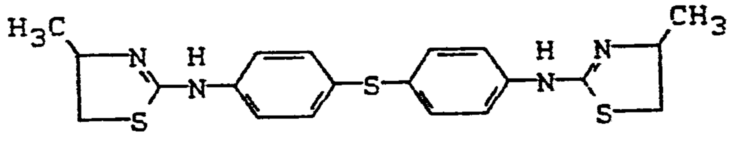

10 g (0,033 mol) Bis(4-isothiocyanatophenyl)sulfid werden in 100 ml Dichlormethan gelöst, auf 0 - 5°C abgekühlt und tropfenweise mit 5,0 g ≡ 5,2 ml (0,066 mol) 2-Amino-1-hydroxypropan versetzt. Man rührt noch ca 1 h bei 0 - 5°C nach, dampft im Vakuum zur Trockene ein und suspendiert den Rückstand in konz. Salzsäure. Die Suspension erhitzt man 1 h am Rückfluß, wobei sich langsam eine klare Lösung bildet. Nach dem Abkühlen fällt man das Produkt unter Rühren durch vorsichtigen Zusatz einer konzentrierten Ammoniaklösung aus; ggfs. Umfällen mit Salzsäure / Ammoniak.

Ausbeute: 1,6 g (11,7% der Theorie)

Schmp. : 180°C (Zers.)

Beispiel 24

Bis[4-(5,6-dihydro-1,3-thiazin-2-yl)aminophenyl]sulfid

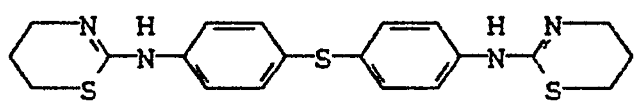

4,2 g (0,014 mol) Bis(4-isothiocyanatophenyl)sulfid werden in 50 ml Dichlormethan gelöst, auf 0 - 5°C abgekühlt und tropfenweise mit 2,1 g ≡ 2,2 ml (0,028 mol) 1-Amino-3-hydroxypropan versetzt. Man rührt noch ca 1 h bei 0 - 5°C nach, dampft im Vakuum zur Trockene ein und suspendiert den Rückstand in konz. Salzsäure. Die Suspension erhitzt man 1 h am Rückfluß, wobei sich langsam eine klare Lösung bildet. Nach dem Abkühlen fällt man

das Produkt unter Rühren durch vorsichtigen Zusatz einer konzentrierten Ammoniaklösung aus; ggfs. Umfällen mit Salzsäure / Ammoniak.
Ausbeute: 5,6 g (96,6% der Theorie)
Schmp. : 172°C (Zers.)

Beispiel 25

4-Tosylamino-4'-nitrodiphenylsulfid

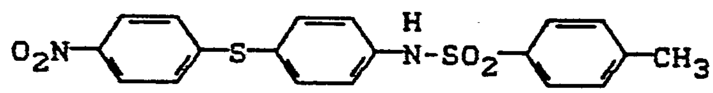

25,1 g (0,1 mol) 4-Amino-4'-nitrodiphenylsulfid werden in 1500 ml Dioxan gelöst und mit 8 ml (0,1 mol) Pyridin versetzt. Zu dieser Lösung tropft man unter Rühren 19,4 g (0,1 mol) Tosylchlorid in 200 ml Dioxan. Dabei sinkt die Temperatur auf 12°C. Man erwärmt über Nacht am Rückfluß. Nach dem Abkühlen dampft man im Vakuum zur Trockene ein, nimmt den Rückstand in ca. 1 l Essigsäureethylester auf und wäscht zweimal mit 2 N Natronlauge. Nach Neutralwaschen und Trocknen dampft man im Vakuum das Lösemittel ab und verrührt den Rückstand mit Diethylether.
Ausbeute: 20,5 g (51,3% der Theorie)
Schmp. : 324°C (Zers.)

0240680

Beispiel 26

4-Tosylamino-4'-aminodiphenylsulfid

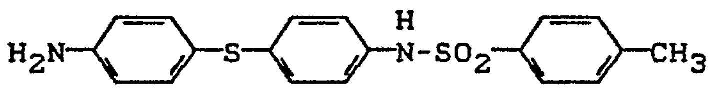

Man löst 18 g (0,045 mol) 4-Tosylamino-4'-nitrodiphenylsulfid in 200 ml Methanol, versetzt mit 50 ml einer 5%igen Raney-Nickel-Suspension und erwärmt auf 35°C. Die Reduktion erfolgt nach vorsichter Zugabe ($N_2$-Entwicklung) von 5,6 g (0,112 mol) Hydrazinhydrat. Man läßt noch 30 min bei 35°C nachreagieren und filtriert vom Unlöslichen ab. Nach dem Eindampfen im Vakuum suspendiert man in Wasser und extrahiert mit Dichlormethan. Daraus gewinnt man nach Trocknen und Einrotieren das Produkt.

Ausbeute: 12,5 g (75,3% der Theorie)

Schmp. : 135°C

Beispiel 27

4-Tosylamino-4'-isothiocyanatodiphenylsulfid

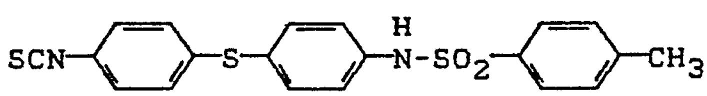

2,5 ml (0,031 mol) Thiophosgen werden in 100 ml Dioxan gelöst, auf 0 - 5°C abgekühlt und 10 g (0,027 mol) 4-Tosylamino-4'-aminodiphenylsulfid, gelöst in 400 ml Dioxan, langsam zugetropft. Anschließend versetzt man mit 6,2 g ≡ 8,5 ml (0,062 mol) Pyridin und läßt unter Rühren noch 3 h bei 5 - 10°C reagieren. Man filtriert die Reaktionslösung über Kieselgel, dampft auf ein kleines Volumen ein und trennnt säulenchromatographisch (Kieselgel 60, Dichlormethan : Methanol = 100 : 1 als Laufmittel). Man erhält neben 1,0 g 4,4'-Di-isothiocyanatodiphenylsulfid das gewünschte Produkt.

Ausbeute: 5,7 g (51,4% der Theorie)

Fp. : 183°C (Zers.)

Beispiel 28

4-($\Delta^2$-Thiazolin-2-yl)amino-4'-aminodiphenylsulfid

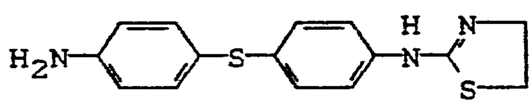

Man löst 2,8 g (0,0068 mol) 4-Tosylamino-4'-isothiocyanatodiphenylsulfid in 50 ml Dichlormethan, kühlt auf 0 - 5°C ab und versetzt tropfenweise unter Rühren mit 0,4 g ≡ 0,4 ml (0,061 mol) Ethanolamin. Man läßt noch 1 h bei Raumtemperatur nachreagieren, dampft im Vakuum zur Trokkene ein und suspendiert den Rückstand in konz. Salzsäure. Nach ca. 1 h Rückfluß erhält man eine ölige und eine wäßrige Phase. Nach Abtrennen alkalisiert man die wäßrige Phase und erhält ein farbloses Produkt.

Ausbeute: 250 mg (12,6% der Theorie)

Schmp. : 148°C

Beispiel 29

4-($\Delta^2$-Thiazolin-2-yl)amino-4'-tosylaminodiphenylsulfid

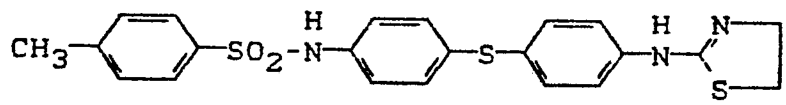

Das abgetrennte Öl aus 4-($\Delta^2$-Thiazolin-2-yl)amino-4'-aminodiphenylsulfid (Beispiel 28) wird in Methanol aufgenommen und unter Rühren mit Ammoniaklösung alkalisiert. Das ausgefallene Produkt wird in Dichlormethan aufgenommen, mit Wasser gewaschen, getrocknet und im Vakuum zur Trockene eingedampft.
Ausbeute: 2,2 g (65,7% der Theorie)
Schmp. : >220°C (Zers.)

Beispiel 30

4-(3-Methyl-thiazolidin-2-yl)imino-4'-tosylaminodiphenylsulfid

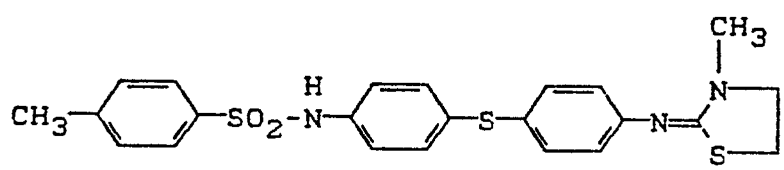

2,8 g (0,0068 mol) 4-Tosylamino-4'-isothiocyanatodiphenylsulfid (Beispiel 27) werden in 50 ml Dichlormethan gelöst, auf 0 - 5°C abgekühlt und tropfenweise mit 0,5 g

≡ 0,55 ml (0,0068 mol) N-Methylethanolamin versetzt. Man läßt noch 1 h bei Temperatur nachreagieren und saugt dann den ausgefallenen Niederschlag ab. Das noch feuchte Produkt suspendiert man in 50 ml konz. Salzsäure und erhitzt 1 h am Rückfluß. Das ausgefallene gelbe Öl trennt man von der wäßrigen Phase ab, nimmt in Methanol auf und alkalisiert unter Rühren mit Ammoniaklösung. Man engt im Vakuum zur Trockene ein, nimmt den Rückstand in Dichlormethan auf, wäscht mit Wasser, trocknet die Dichlormethanphase und engt zur Trockne ein .

Ausbeute: 1,8 g (56,5% der Theorie)

Fp. : >220°C (Zers.)

Beispiel 31

4,4'-(3,3'-Diacetylthiazolidin-2-yl)diimino-diphenylsulfid

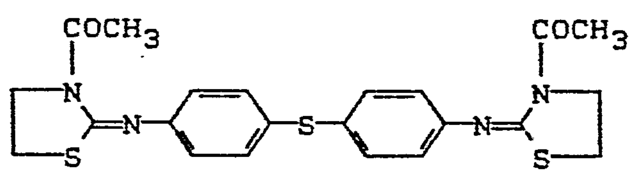

Verfahrensvariante A:

3,0 g (0,0077 mol) Bis[4-($\Delta^2$-thiazolin-2-yl)aminophenyl]sulfid (Beispiel 2) werden in 50 ml getrocknetem Dimethylformamid gelöst und mit 0,46 g (0,0152 mol) Natriumhydrid versetzt. Nachdem man 1 h im Ultraschallbad hat reagieren lassen, tropft man bei Raumtemperatur 1,2 g ≡ 1 ml (0,015 mol) frisch destilliertes Acetylchlorid zu und läßt 24 h nachreagieren. Dann dampft man

 0240080

das Reaktionsgemisch im Vakuum zur Trockene ein, suspendiert den Rückstand in Dichlormethan und wäscht zweimal mit Wasser. Nach Einrotieren der organischen Phase erhält man ein gelbes Öl, das mono- und diacetyliertes Ausgangsprodukt enthält. Nach säulenchromatographischer Trennung (Kieselgel 60, Dichlormethan : Methanol = 100:1) erhält man das gewünschte Diacetylprodukt.

Ausbeute: 1,5 g (41% der Theorie)

Fp. : 120 - 122°C

Verfahrensvariante B

3,0 g (0,0077 mol) Bis[4-($\Delta^2$-thiazolin-2-yl)aminophenyl]sulfid (Beispiel 2) werden in 100 ml frischem Acetanhydrid gelöst, 24 h bei Raumtemperatur gerührt und auf ca. 300 ml Eiswasser gegeben. Man extrahiert dreimal mit Dichlormethan, wäscht mit Wasser, trocknet die organische Phase und dampft zur Trockene ein.

Ausbeute: 2,0 g (54,8% der Theorie)

Fp. : 120 - 122°C

Beispiel 32

4-(3-Methyl-thiazolidin-2-yl)imino-4'-amino-diphenylsulfid

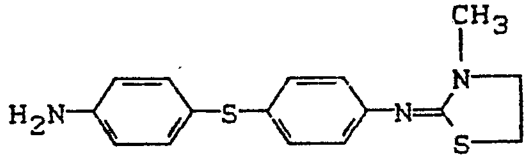

Die vom gelbem Öl aus 4-(3-Methylthiazolidin-2-yl)-imino-4'-tosylaminodiphenylsulfid (Beispiel 30) befreite wäßrige Phase wird mit Ammoniakwasser alkalisch gestellt, wobei das gewünschte Amin ausfällt.
Ausbeute: 0,18 g (9,4% der Theorie)
Fp. : 131 - 133°C

Beispiel 33

4-Chlor-4'-isothiocyanatodiphenylsulfid

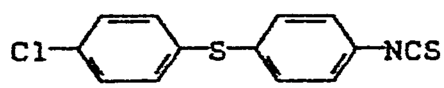

4,2 ml (0.054 mol) Thiophosgen werden in 50 ml Dichlormethan vorgelegt, mit 200 ml Wasser unterschichtet und auf 0 - 5°C abgekühlt. Unter kräftigem Rühren werden 12,8 g (0,054 mol) 4-Chlor-4'-amino-diphenylsulfid in 200 ml Dichlormethan und 4,32 g NaOH in 50 ml Wasser zugegeben. Man läßt anschließend noch 2 h bei 5°C nachreagieren. Dann trennt man die organische Phase ab, trocknet ein und dampft im Vakuum zur Trockene ein. Den Rückstand trennt man säulenchromatographisch (Kieselgel 60, Cyclohexan)
Ausbeute: 14,5 g (98% der Theorie)
IR ($CHCl_3$): NCS-Bande bei 2020 $cm^{-1}$

Beispiel 34

4-($\Delta^2$-Thiazolin-2-yl)amino-4'-chlor-diphenylsulfid-hydrochlorid

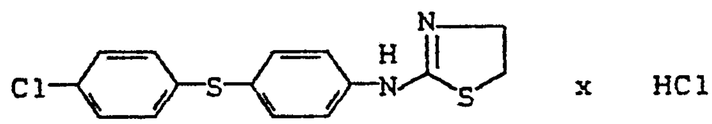

5,0 g (0,018 mol) 4-Chlor-4'-isothiocyanatodiphenylsulfid werden in 100 ml Dichlormethan gelöst, auf 0 -5°C abgekühlt und 1,1 g ≡ 1,1 ml (0,018 mol) Ethanolamin zugetropft. Man läßt 30 min nachrühren, filtriert den ausgefallenen Thioharnstoff ab und suspendiert das feuchte Produkt in konz. Salzsäure. Nach 1 h Erhitzen am Rückfluß trennt man das Öl ab. Nach einiger Zeit kristallisiert daraus das Hydrochlorid aus.
Ausbeute: 4,4 g (77,8% der Theorie)
Fp. : 162°C (Zers.)

Beispiel 35

4-Methyl-4'-isothiocyanatodiphenylsulfid

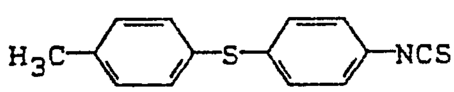

33,8 g ≡ 22,4 ml (0,3 mol) Thiophosgen werden in 100 ml Dichlormethan vorgelegt, mit 400 ml Wasser unterschichtet und 61,5 g (0,3 mol) 4-Amino-4'-methyldiphenylsulfid in 300 ml Dichlormethan unter kräftigem Rühren zugetropft. Man läßt 1 h nachreagieren, trennt die organische Phase ab, trocknet ein und dampft im Vakuum zur Trockene ein. Den Rückstand trennt man säulenchromatographisch (Kieselgel 60, Cyclohexan).
Ausbeute: 58,6 g (76 % der Theorie).

Beispiel 36

4-($\Delta^2$-Thiazolin-2-yl)amino-4'-methyl-diphenylsulfid

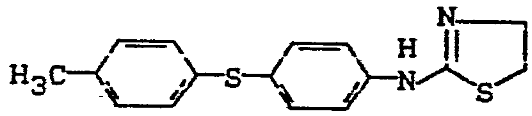

18,0 g (0,07 mol) 4-Methyl-4'-isothiocyanatodiphenylsulfid werden in 250 ml Dichlormethan gelöst, auf 5°C abgekühlt und unter Rühren mit 4,3 g ≡ 4,5 ml Ethanolamin versetzt. Nach 30 min Nachreaktion saugt man den Thioharnstoff ab, suspendiert ihn in konz. Salzsäure und erhitzt 1 h am Rückfluß. Man trennt das ausgefallene Öl ab, nimmt es mit Methanol auf und fällt das gewünschte Produkt mit Ammoniak aus.
Ausbeute: 8,1 g (38,6 % der Theorie)
Fp. : 115°C

Beispiel 37

4-(5,6-Dihydro-thiazin-2-yl)amino-4'-methyl-diphenylsulfid Hydrochlorid

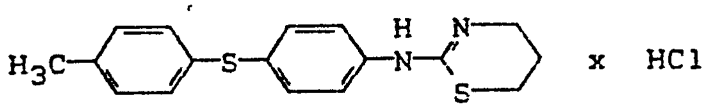

18,0 g (0,07 mol) 4-Methyl-4'-isothiocyanatodiphenylsulfid (Beispiel 35) werden in 250 ml Dichlormethan gelöst, auf 0 bis 5°C abgekühlt und 5,3 g ≡ 5,3 ml 3-Aminopropanol zugetropft. Man läßt 30 min nachrühren, filtriert den Thioharnstoff ab und cyclisiert durch Kochen mit konz. Salzsäure. Das ausgefallene Öl nimmt man in Methanol auf und fällt das gewünschte Produkt mit Ammoniak aus.

Ausbeute: 21,3 g (97% der Theorie)

Fp. : 85 - 87°C

Beispiel 38

4-Methyl-4'-(3-methylthiazolidin-2-yl)imino-diphenylsulfid

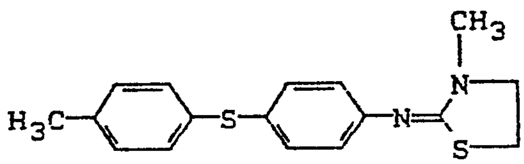

18,0 g (0,07 mol) 4-Methyl-4'-isothiocyanatodiphenylsulfid (Beispiel 35) werden in 250 ml Dichlormethan gelöst, auf 0 bis 5°C abgekühlt und 5,3 g ≡ 5,5 ml N-Methylethanolamin zugetropft. Man läßt 30 min nachreagieren, filtriert den Thioharnstoff ab und cyclisiert durch Kochen mit konzentrierter Salzsäure. Das ausgefallene Öl nimmt

man in Methanol auf und fällt das gewünschte Produkt mit Ammoniak aus.

Ausbeute: 18,3 g (83,3% der Theorie)

Fp. : 59 - 61°C

Beispiel 39

4-Amino-4-thiazol-2-yl-amino-diphenylsulfid

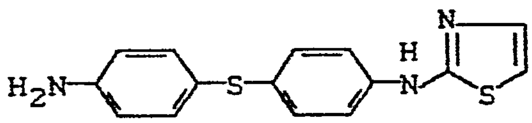

Man löst 4,0 g (0,012 mol) 4-Nitro-4'-thiazol-2-yl-aminodiphenylsulfid (Beispiel 48) und 1,5 g (0,03 mol) Hydrazinhydrat in 50 ml Methanol/Tetrahydrofuran (1:1) und gibt unter Rühren bei Raumtemperatur Raney-Nickel in Methanol in kleinen Portionen hinzu. Unter Gasentwicklung erwärmt sich das Reaktionsgemisch leicht. Anschließend erwärmt man 3 h am Rückfluß, filtriert vom Unlöslichen ab, engt das Filtrat im Vakuum zur Trockene ein und trennt den Rückstand säulenchromatographisch (Kieselgel 60, Dichlormethan : Tetrahydrofuran = 9:1).

Ausbeute: 3,3 g (92% der Theorie)

Fp. : 119°C

Beispiel 40

4-Isothiocyanato-4'-thiazol-2-yl-amino-diphenylsulfid

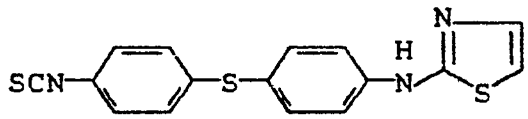

Man löst 1,3 g (0,01 mol) Thiophosgen in 10 ml Dichlormethan, unterschichtet mit 20 ml Wasser und tropft 3,0 g (0,01 mol) 4-Amino-4-thiazol-2-yl-aminodiphenylsulfid (Beispiel 39) in 50 ml Dichlormethan / Tetrahydrofuran (1:1) hinzu. Anschließend tropft man unter kräftigem Rühren bei Raumtemperatur 10 ml 2 N Natronlauge hinzu und rührt noch 2 h nach. Die dunkelrote organische Phase wird abgetrennt und säulenchromatographiert (Kieselgel 60, Dichlormethan : Cyclohexan = 1:1).
Ausbeute: 3,2 g (93,6% der Theorie)
Fp : 134° C

Beispiel 41

4-($\Delta^2$-Thiazolin-2-yl)-amino-4'-thiazol-2-yl-amino-diphenylsulfid

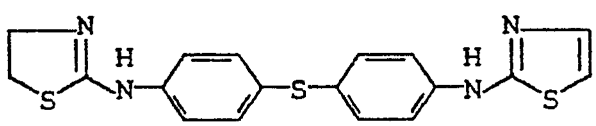

3,2 g (0,0094 mol) des Isothiocyanats aus Beispiel 40 werden in 50 ml Dichlormethan gelöst und tropfenweise mit 0,6 g ≡ 0,6 ml (0,0094 mol) Ethanolamin versetzt. Die rote Farbe des Sulfids verschwindet schlagartig. Man dampft zur Trockene ein, suspendiert den Rückstand in konz. Salzsäure und erhitzt 1 h am Rückfluß. Der Ansatz wird mit Wasser verdünnt und mit 2 N Natronlauge das Endprodukt ausgefällt. Anschließend wird säulenchromatographisch getrennt (Kieselgel 60, Eisessig / Methanol / Dichlormethan = 10/5/85).

Ausbeute: 2,2 g (61% der Theorie)

Fp. : 182° C

Beispiel 42

4-Nitro-4'-isothiocyanato-diphenylsulfid

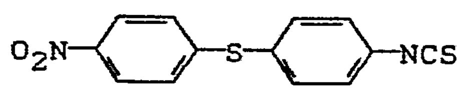

12 g ≡ 7,9 ml (0,1 mol) Thiophosgen werden in 100 ml Dichlormethan gelöst, mit 200 ml Wasser unterschichtet und bei 0 - 5° C unter kräftigem Rühren mit einer Lösung von 24,6 g (0,1 mol) 4-Amino-4'-nitro-diphenylsulfid in 250 ml Dichlormethan und mit 8 g (0,2 mol) NaOH in 50 ml Wasser versetzt. Man läßt noch 2 h bei 0 - 5° C nachreagieren, trennt die organische Phase ab, dampft sie zur Trockene ein und säulenchromatographiert den Rückstand (Kieselgel 60, Cyclohexan).

Ausbeute: 28,1 g (99,6% der Theorie)

Fp : 109° C

Beispiel 43

4-Nitro-4'-($\Delta^2$-thiazolin-2-yl)amino-diphenylsulfid

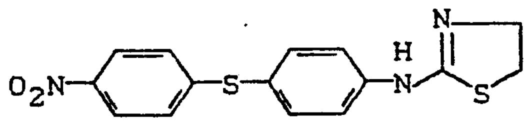

28 g (0,1 mol) des obigen Isothiocyanates (Beispiel 42) werden in 300 ml Dichlormethan gelöst, auf 0 - 5° C abgekühlt, und 5,9 g ≡ 5,9 ml (0,1 mol) Ethanolamin zugetropft. Nach 1 h Weiterrühren saugt man den Thioharnstoff ab, suspendiert den Rückstand in konz. Salzsäure und erhitzt 1 h am Rückfluß. Das ausgefallene Öl nimmt man in Methanol auf und fällt mit Ammoniak das Produkt aus.

Ausbeute: 28 g (84,6% der Theorie)

Fp. : 178° C

Beispiel 44

4-Nitro-4'-(3-trifluormethylphenyl)sulfonamino-diphenylsulfid

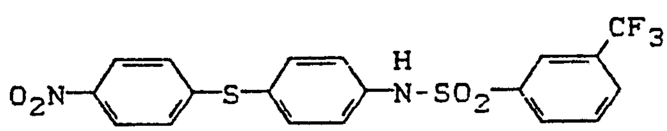

25,1 g (0,1 mol) 4-Amino-4'-nitrodiphenylsulfid werden in 1500 ml Dioxan gelöst, mit 8 ml (0,1 mol) Pyridin versetzt, und unter Rühren werden 24,5 g (0,1 mol) 3-Trifluormethylbenzolsulfonylchlorid zugetropft. Man läßt 48 h bei Raumtemperatur nachreagieren, dampft im Vakuum zur Trockene ein, nimmt den Rückstand in Essigester auf, wäscht mit Wasser neutral, trocknet und dampft das Lösemittel ab.

Ausbeute: 24,5 g (54% der Theorie)

Fp. : 113°C

Beispiel 45

4-Amino-4'-(3-trifluormethylphenyl)sulfonylamino-diphenylsulfid

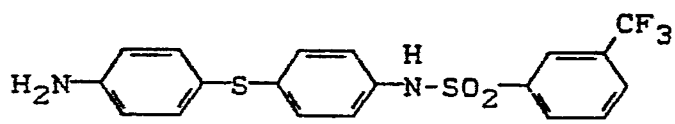

24,4 g (0,054 mol) der Nitroverbindung aus Beispiel 44 werden in 300 ml Methanol gelöst, mit 6,7 g ≡ 6,5 ml (0,135 mol) Hydrazinhydrat und nach und nach mit 25 ml 5%iger Raney-Nickel-Suspension versetzt und am Rückfluß erwärmt. Man läßt noch 30 min nachreagieren, bis sich kein $N_2$ mehr entwickelt. Nach Abfiltrieren des Unlöslichen engt man das Filtrat im Vakuum zur Trockene ein und nimmt den Rückstand in Dichlormethan auf. Man wäscht zweimal mit Wasser und extrahiert mit Salzsäure. Nach Alkalisieren mit Natronlauge extrahiert man mit Dichlormethan, trocknet mit $Na_2SO_4$ und dampft im Vakuum zur

Trockene ein. Der Rückstand wird säulenchromatographiert (Kieselgel 60, Dichlormethan).
Ausbeute: 6,3 g (27,5% der Theorie)
Fp. : 133° C

Beispiel 46

4-Isothiocyanato-4'-(3-trifluormethylphenyl)sulfonylamino-diphenylsulfid

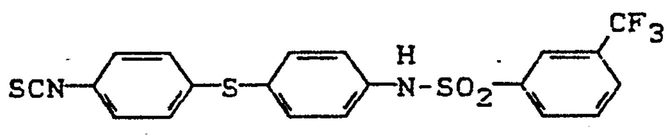

1,59 g ≡ 1,1 ml (0,013 mol) Thiophosgen werden in 50 ml Dichlormethan gelöst, mit 20 ml Wasser unterschichtet und auf 0 - 5° C abgekühlt. Dazu tropft man unter kräftigem Rühren 5,7 g (0,013 mol) des Amins aus Beispiel 45 in 100 ml Dichlormethan. Nach 30 min Nachreagieren wird die organische Phase abgetrennt, getrocknet und im Vakuum zur Trockene eingedampft. Anschließend erfolgt eine säulenchromatographische Trennung (Kieselgel 60, Dichlormethan).
Ausbeute: 5,2 g (85,8% der Theorie)
Fp. : 93° C

Beispiel 47

4-($\Delta^2$-Thiazolin-2-yl)amino-4'-(3-trifluormethylphenyl)-sulfonylamino-diphenylsulfid

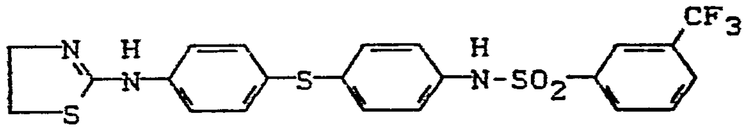

5,2 g (0,011 mol) des Isothiocyanats aus Beispiel 46 werden in 100 ml Dichlormethan gelöst und bei 0 - 5°C unter Rühren mit 0,08 g ≡ 0,08 ml (0,011 mol) Ethanolamin versetzt. Man läßt 30 min nachreagieren, filtriert den Thioharnstoff ab, suspendiert in konz. Salzsäure und erhitzt 1 h zum Sieden. Das ausfallende Öl trennt man von der wäßrigen Phase ab und fällt das Thiazolin mit Ammoniak aus.

Ausbeute: 5,1 g (91,1% der Theorie)

Fp. : 182°C

## Beispiel 48

N-[4-(4-Nitrophenylthio)phenyl]thioharnstoff

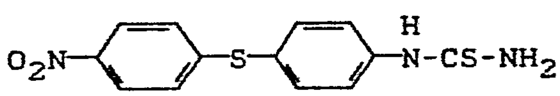

24,6 g (0,1 mol) 4-Nitro-4'-aminodiphenylsulfid werden in 150 ml Dichlormethan gelöst, mit 100 ml Wasser unterschichtet und unter kräftigem Rühren mit 12,7 g ≡ 8,4 ml (0,11 mol) Thiophosgen in 50 ml Dichlormethan versetzt. Man tropft 50 ml 2 N Natronlauge hinzu, wobei leichte Erwärmung auftritt (30 - 35°C). Nach 3 h Rühren bei Raumtemperatur trennt man die organische Phase ab

und versetzt mit 25%iger Ammoniaklösung. Man rührt das Reaktionsgemisch über Nacht und filtriert den Thioharnstoff ab.
Ausbeute: 25,1 g (83% der Theorie)
Fp. : 149° C

Beispiel 49

4-Nitro-4'-thiazol-2-yl-amino-diphenylsulfid

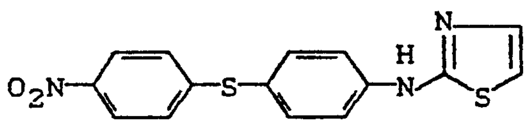

Man suspendiert 6,1 g (0,002 mol) des Thioharnstoffs aus Beispiel 48 in 20 ml Wasser und gibt 5,8 g ≡ 5 ml (0,04 mol) 1-Chlordiethylether (85%ig) hinzu. Dabei entsteht eine klare Lösung, die sich leicht erwärmt. Man erhitzt noch 1 h auf 80° C, läßt erkalten und neutralisiert mit $NaHCO_3$-Lösung. Der ausgefallene Niederschlag wird abgesaugt und getrocknet.
Ausbeute: 4,7 g (72% der Theorie)
Fp. : 159° C

Analog Beispiel 49 wurden hergestellt:

Beispiel 50

Bis[4,4'-(bisthiazol-2-yl)amino]diphenylsulfid

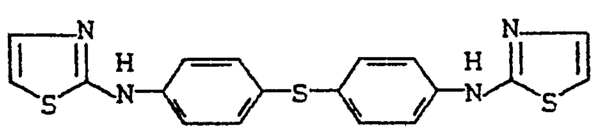

Ausbeute: 70% der Theorie (bezogen auf $(H_2NCSNH-C_6H_4)_2S$)

Beispiel 51

4-(Thiazol-2-yl)amino-diphenylsulfid

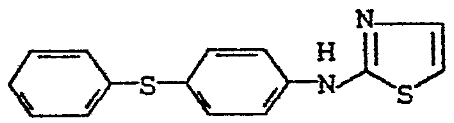

Ausbeute: 71% der Theorie (bezogen auf $H_5C_6-S-C_6H_5-NHCSNH_2$)

Fp. : 128° C

Beispiel 52

4-(4-Methyl-thiazol-2-yl)amino-diphenylsulfid

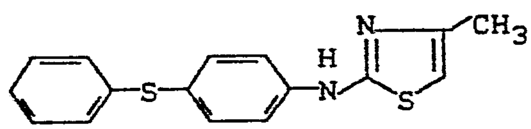

Ausbeute: 64% der Theorie (bezogen auf $H_5C_6-S-C_6H_5-NHCSNH_2$)

Fp. : 112° C

Analog Beispiel 3 wurden hergestellt:

Beispiel 53

Bis[4-($\Delta^2$-thiazolin-2-yl)aminophenyl]sulfid Lactat

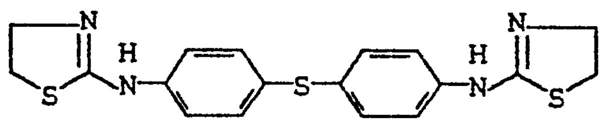

x 1,5 $C_5H_6O_3$

Beispiel 54

Bis[4-($\Delta^2$-thiazolin-2-yl)aminophenyl]sulfid Sulfat

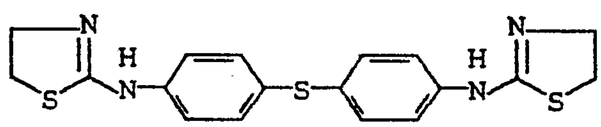

x 1,5 $H_2SO_4$

Beispiel 55

Bis[4-($\Delta^2$-thiazolin-2-yl)aminophenyl]sulfid Citrat

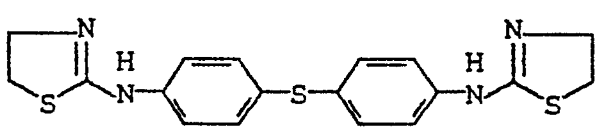

x 2/3 $C_6H_8O_7$

## Anwendungsbeispiel

Die pharmakologische Wirkung der erfindungsgemäßen Substanzen wurde durch folgende Methode bestimmt:

Als Maß für die Lipoxygenase-Aktivität von PMN der Ratte wird das nach Stimulation mit Ca-Ionophor gebildete Leukotrien $B_4$ ($LTB_4$) mittels reversed phase HPLC nach Borgeat et al. Proc. Natl. Acad. Sci. USA 76, 215 (1982) bestimmt.

In Tabelle 1 sind beispielsweise die nach diesem Test ermittelten $IC_{50}$-Werte der folgenden Verbindungen aufgeführt.

Tabelle 1

| Bsp.-Nr. | $IC_{50}$-Werte [g/ml] |
|---|---|
| 2 | $1.35 \cdot 10^{-7}$ |
| 3 | $3.0 \cdot 10^{-7}$ |
| 4 | $2.7 \cdot 10^{-7}$ |
| 10 | $7.5 \cdot 10^{-8}$ |
| 12 | $2.8 \cdot 10^{-8}$ |
| 17 | $1.1 \cdot 10^{-7}$ |
| 22 | $5.1 \cdot 10^{-8}$ |
| 29 | $6.6 \cdot 10^{-8}$ |

Patentansprüche

1. Diarylsulfidderivate der Formel

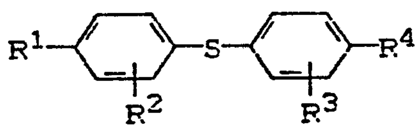 (I),

in welcher

$R^1$ - für einen Thiazolamino-Rest der Formeln

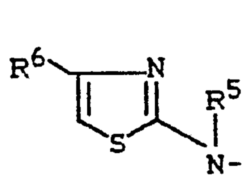 , 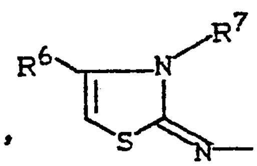 , 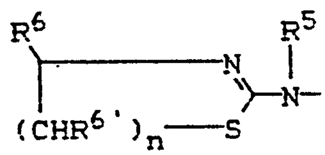

oder 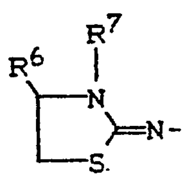 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Acyl oder Aryl steht, und

n - für eine Zahl 1 oder 2 steht,

$R^2$, $R^3$ - gleich oder verschieden sind und

für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

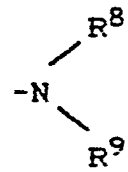 stehen,

worin

$R^8$, $R^9$ - gleich oder verschieden sind und

- für Wasserstoff, Alkyl, Aryl, Aralkyl, Acyl, Trifluoracetyl, Alkylsulfonyl, Arylsulfonyl, Trifluormethylphenylsulfonyl oder Tolylsulfonyl stehen,

und

$R^4$ - eine der oben genannten Bedeutungen von $R^1$ hat,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

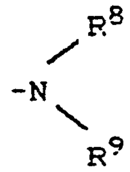 steht,

wobei

$R^8$, $R^9$ die oben angegebene Bedeutung haben,

und deren Salze.

2. Diarylsulfidderivate nach Anspruch 1, worin

$R^1$ - für einen Thiazolamino-Rest der Formeln

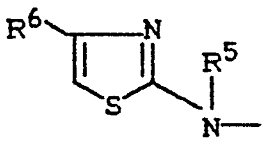 , 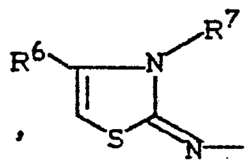 , 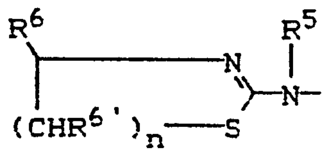

oder 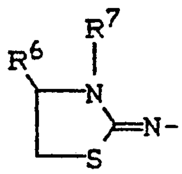 steht,

worin

$R^5$ - für Wasserstoff, Niederalkyl, Benzyl, Benzoyl oder Acetyl steht,

$R^6$, $R^{6'}$ - gleich oder verschieden sind und für Wasserstoff, Niederalkyl oder Phenyl steht,

$R^7$ - für Niederalkyl, Cyclohexyl, Benzyl, Acetyl, Benzoyl oder Phenyl steht, und

n - für eine Zahl 1 oder 2 steht,

$R^2$, $R^3$ - gleich oder verschieden sind und

- für Wasserstoff, Niederalkyl, Niederalkenyl, Niederalkoxy, Niederalkylthio, Halogenniederalkyl, Halogenniederalkoxy, Trifluormethylthio, Phenyl, Benzyl, Phenoxy, Benzyloxy, Niedrigalkylcarbonyl, Benzoyl, Carboxy, Niedrigalkoxycarbonyl, Carboxyniedrigalkyl, Niedrigalkoxycarbonylniedrigalkyl, Nitro, Cyano, Fluor, Chlor, Brom oder für die Gruppe der Formel

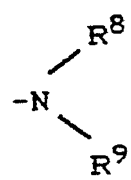

stehen,

worin

$R^8$, $R^9$ - gleich oder verschieden sind und

- für Wasserstoff, Niedrigalkyl, Phenyl, Benzyl, Niedrigalkylcarbonyl, Benzoyl, Trifluoracetyl, Niederalkylsulfonyl, Phenylsulfonyl, Trifluormethylphenylsulfonyl oder Tolylsulfonyl stehen,

0240680

und

$R^4$ - eine der oben genannten Bedeutungen von $R^1$ hat, oder

- für Wasserstoff, Niederalkyl, Niederalkenyl, Niederalkoxy, Niederalkylthio, Halogenniederalkyl, Halogenniederalkoxy, Trifluormethylthio, Phenyl, Benzyl, Phenoxy, Benzyloxy, Niederalkylcarbonyl, Benzoyl, Carboxy, Niederalkoxycarbonyl, Carboxyniederalkyl, Niederalkoxyniederalkyl, Nitro, Cyano, Fluor, Chlor, Brom oder für eine Gruppe der Formel

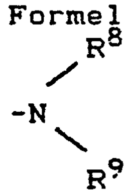

steht,

worin

$R^8$, $R^9$ die oben angegebene Bedeutung hat,

und deren Salze.

3. Diarylsulfidderivate nach den Ansprüchen 1 und 2,

worin

$R^1$-für einen Thiazolaminorest der Formeln

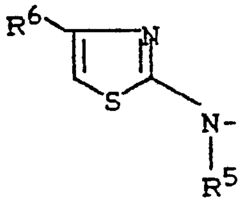

,

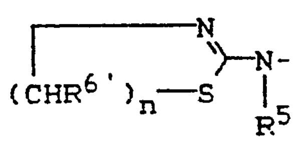

oder

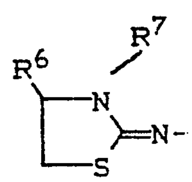

steht, worin

$R^5$ - für Wasserstoff, Methyl, Ethyl, Propyl, Isopropyl, Benzyl oder Acetyl steht, und

$R^6$, $R^{6'}$ - gleich oder verschieden sind und für Wasserstoff, Methyl, Ethyl, Propyl, Isopropyl, Butyl, Isobutyl oder Phenyl steht,

und

$R^7$ - für Methyl, Ethyl oder Acetyl steht,

$R^2$, $R^3$ - gleich oder verschieden sind und für Wasserstoff, Methyl, Ethyl, Propyl, Isopropyl, Allyl, Methoxy, Methylthio, Trifluormethyl, Trifluormethoxy, Difluormethyl, Trifluormethylthio, Acetyl, Carboxy, Carboxymethyl, Methoxycarbonyl, Ethoxycarbonyl, Methoxycarbonylmethyl, 1-Methoxycarbonyl-ethyl,2-Ethoxycarbonyl-ethyl, 2-Ethoxycarbonyl-ethyl, Cyano, Fluor, Chlor oder Brom stehen,

$R^4$ - eine der oben genannten Bedeutungen von $R^1$ hat, oder
- für Wasserstoff, Methyl, Ethyl, Propyl, Isopropyl, Butyl, tert-Butyl, Methoxy, Methylthio, Trifluormethyl, Trifluormethoxy, Trifluormethylthio, Acetyl, Carboxy, Methoxycarbonyl, Ethoxycarbonyl, Carboxymethyl, Methoxycarbonylmethyl, Ethoxycarbonyl-methyl, 1-Methoxycarb-

onylethyl, 2-Methoxycarbonylethyl, 1-Ethoxycarbonylethyl, 2-Ethoxycarbonylethyl, Nitro, Cyano, Fluor, Chlor, Brom oder eine Gruppe der Formel

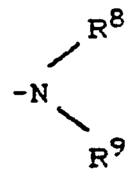 steht,

worin

$R^8$, $R^9$ - gleich oder verschieden sind und
- für Wasserstoff, Methyl, Ethyl, Propyl, Isopropyl, Phenyl, Benzyl, Acetyl, Trifluoracetyl, Methylsulfonyl, Phenylsulfonyl oder Trifluormethylphenylsulfonyl, Toluylsulfonyl stehen,

und deren Salze.

4. Diarylsulfidderivate der Formel

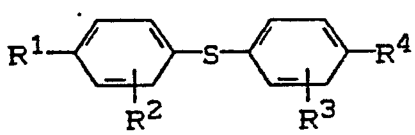 (I),

in welcher

$R^1$ - für einen Thiazolamino-Rest der Formeln

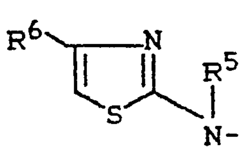 , 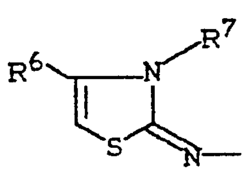 , 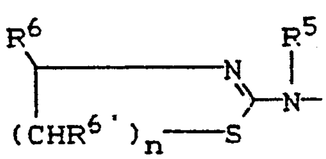

oder 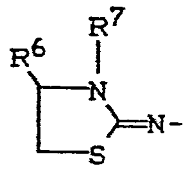 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht, und

n - für eine Zahl 1 oder 2 steht,

$R^2$, $R^3$ - gleich oder verschieden sind und

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

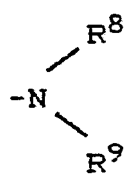 stehen,

worin

$R^8$, $R^9$ - gleich oder verschieden sind und

- für Wasserstoff, Alkyl, Aryl, Aralkyl, Acyl, Trifluoracetyl, Alkylsulfonyl, Arylsulfonyl,

0240680

Trifluormethylphenylsulfonyl oder Tolylsulfonyl stehen,

und

$R^4$ - eine der oben genannten Bedeutungen von $R^1$ hat,

oder

- für Wasserstoff, Alkyl, Alkenyl, Cycloalkyl, Alkoxy, Alkylthio, Halogenalkyl, Halogenalkoxy, Halogenalkoxythio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

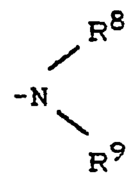

steht,

wobei
$R^8$, $R^9$ die bereits angegebene Bedeutung haben,

und deren Salze zur therapeutischen Behandlung.

5. Verfahren zur Herstellung von Diarylsulfidderivaten der Formel

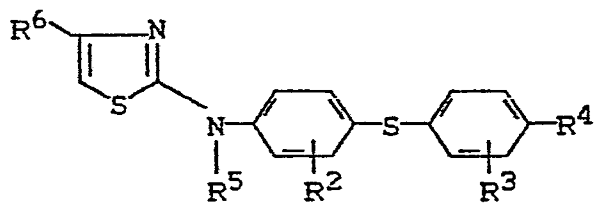 (Ia)

in welcher

$R^5$ - für Wasserstoff, Alkyl, Aralkyl oder Acyl steht und

$R^6$ - für Wasserstoff, Alkyl oder Aryl steht,

$R^2$, $R^3$ - gleich oder verschieden sind und
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

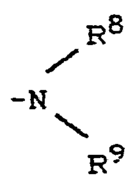 stehen,

worin
$R^8$, $R^9$ - gleich oder verschieden sind und

- für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formeln

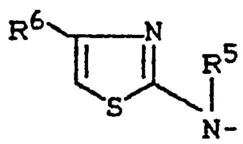 , 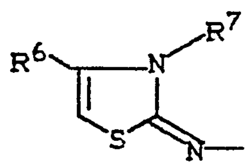 , 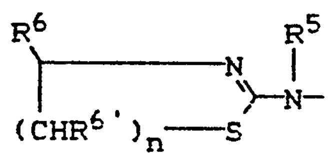

oder 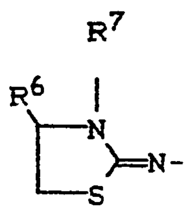 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy; Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

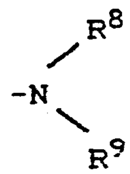

steht,

worin

$R^8$, $R^9$ - die oben angegebene Bedeutung haben,

dadurch gekennzeichnet, daß man Halogenverbindungen der allgemeinen Formel (II)

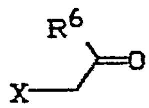

(II),

in welcher

$R^6$ - die oben angegebene Bedeutung hat und

X - für Chlor, Brom oder Iod, bevorzugt für Chlor oder Brom steht,

mit Thioharnstoffen der allgemeinen Formel (III)

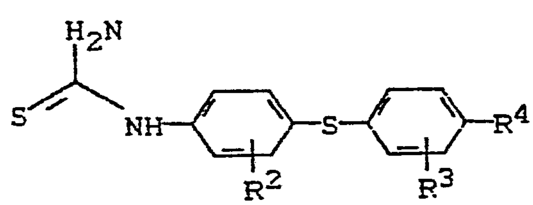

(III),

in welcher

$R^2$ - $R^4$ die oben angegebene Bedeutung haben,

gegebenenfalls in Wasser und/oder inerten organischen Lösungsmitteln, gegebenenfalls in Anwesenheit von Basen umsetzt, gegebenenfalls anschließend anfallende Salze mit Basen in freie Verbindungen überführt und diese gegebenenfalls alkyliert, aralkyliert oder acyliert,

oder daß man Thiazole der allgemeinen Formel (IV)

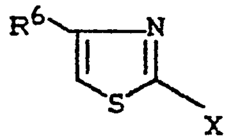

(IV),

in welcher

X - für Chlor, Brom oder Iod, insbesondere für Chlor oder Brom steht, und

$R^6$ - die angegebene Bedeutung hat,

mit Aminen der allgemeinen Formel (V)

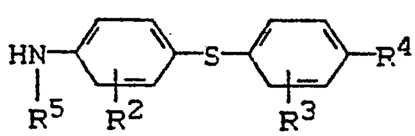

(V),

in welcher

$R^2$ - $R^5$ die oben angegebene Bedeutung haben,

in Wasser und/oder inerten organischen Lösungsmitteln, gegebenenfalls in Anwesenheit eines Katalysators umsetzt, und gegebenenfalls anfallende Salze mit Basen in die freien Verbindungen überführt,

oder daß man Thiophenole der allgemeinen Formel (VI)

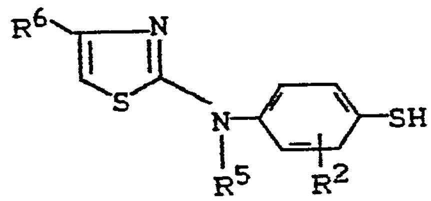

(VI)

in welcher

$R^2$, $R^5$, $R^6$ - die oben angegebene Bedeutung haben,

mit Halogenarylen der allgemeinen Formel (VII)

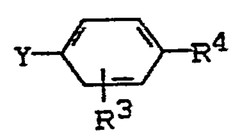

(VII)

in welcher

$R^3$, $R^4$ - die oben angegebene Bedeutung haben und
Y - für Chlor, Brom oder Iod, steht,

gegebenenfalls in Wasser und/oder inerten organischen Lösungsmitteln in Anwesenheit einer Base, gegebenenfalls in Gegenwart eines Katalysators umsetzt.

 0240680

6. Verfahren zur Herstellung von Diarylsulfidderivaten der Formel

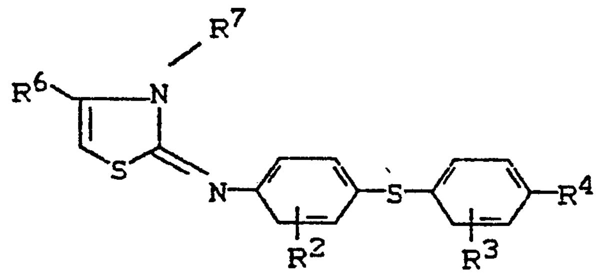

(Ib)

in welcher

$R^6$ - für Wasserstoff, Alkyl, Aralkyl oder Aryl steht,

$R^7$ - für Alkyl, Cycloalkyl, Aralkyl, Aryl oder Acyl steht,

$R^2$, $R^3$ - gleich oder verschieden sind und
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

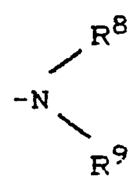

stehen,

worin

$R^8$, $R^9$ - gleich oder verschieden sind und
- für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

$R^4$ - für einen Rest der Formeln

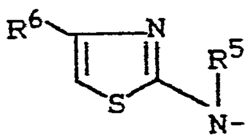 , 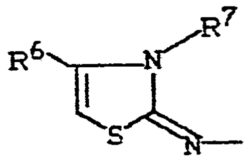 , 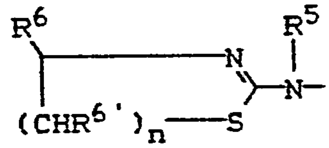

oder 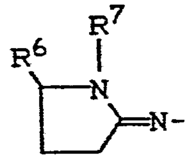 steht,

worin

$R^5$ - für Wasserstoff, Alkyl, Aralkyl oder Acyl steht,

$R^6$, $R^{6'}$ - gleich oder verschieden sind und für Wasserstoff, Alkyl oder Acyl steht,

$R^7$ für Alkyl, Cycloalkyl, Aralkyl, Aryl oder Acyl steht, und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

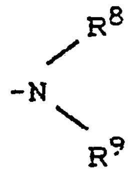

steht,

worin
$R^8$, $R^9$ - die oben angegebene Bedeutung haben,

dadurch gekennzeichnet, daß man Halogenverbindungen der allgemeinen Formel (II)

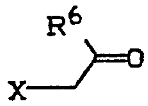

(II),

in welcher

$R^6$ - die angegebene Bedeutung hat und
X - für Chlor, Brom oder Jod steht,

mit substituierten Thioharnstoffen der allgemeinen Formel (IX)

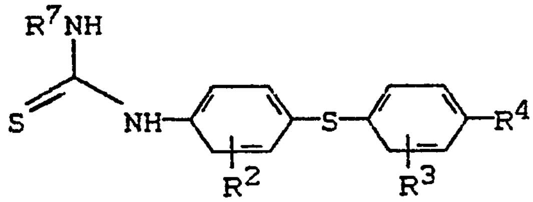

(IX)

in welcher

$R^2$-$R^4$, $R^7$ - die oben angegebene Bedeutung haben,

gegebenenfalls in Wasser und/oder inerten organischen Lösungsmitteln, gegebenenfalls in Anwesenheit einer Base umsetzt, und gegebenenfalls anfallende Salze mit Basen in die freie Verbindung überführt.

7. Verfahren zur Herstellung von Diarylsulfidderivaten der Formel

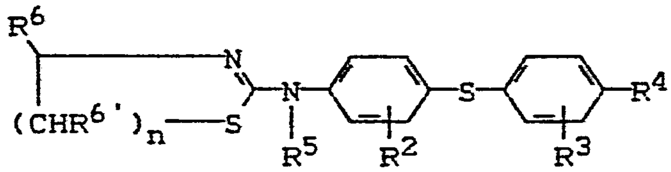

in welcher

$R^5$ - für Wasserstoff, Alkyl oder Acyl steht,
$R^6$, $R^{6'}$ - gleich oder verschieden sind und für Wasserstoff, Alkyl oder Aryl steht,
und
n - für eine Zahl 1 oder 2 steht,

$R^2$, $R^3$ - gleich oder verschieden sind und
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen, oder für eine Gruppe der Formel

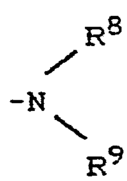 stehen,

worin

$R^8$, $R^9$ - gleich oder verschieden sind und - für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formeln

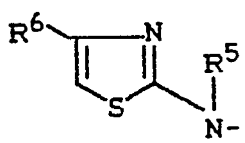 , 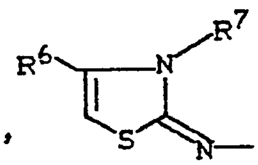 , 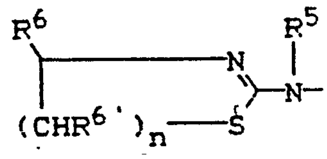

oder 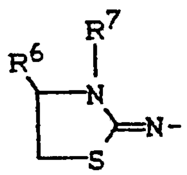 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

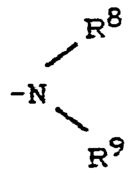

steht,

wobei
$R^8$, $R^9$ die bereits angegebene Bedeutung haben,

dadurch gekennzeichnet, daß man Aminoethanole der Formel (X)

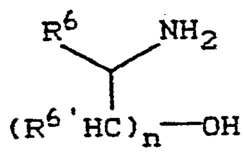

(X)

in welcher

$R^6$, $R^{6'}$ und n die oben angegebene Bedeutung haben mit Senfölen der allgemeinen Formel (XI)

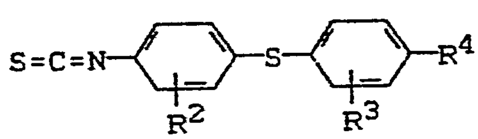

(XI),

in welcher

$R^2$ - $R^4$ die angegebene Bedeutung haben,

in inerten organischen Lösungsmitteln umsetzt, gegebenenfalls aryliert, alkyliert oder acyliert, oder daß man Halogensenföle der allgemeinen Formel XII,

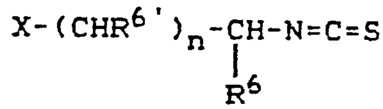

(XII)

in welcher

$R^6$, $R^{6'}$ und n die oben angegebene Bedeutung haben und

X - für Chlor, Brom oder Iod steht,

mit Aminen der allgemeinen Formel (V)

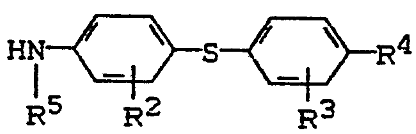

(V)

in welcher

$R^2$ bis $R^5$ die oben angegebene Bedeutung haben,

gegebenenfalls in Anwesenheit von Basen in inerten organischen Lösungsmitteln umsetzt,

oder daß man Thiophenole der allgemeinen Formel XIII

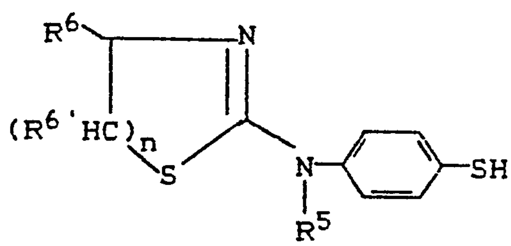

(XIII),

in welcher

$R^2$, $R^5$, $R^6$, $R^{6'}$ und n die angegebene Bedeutung haben,

mit Halogenarylen der allgemeinen Formel (VII),

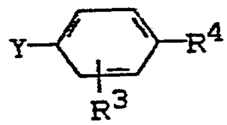

(VII)

in welcher

$R^3$, $R^4$ - die oben angegebene Bedeutung haben und
Y - für Chlor, Brom oder Iod steht,

gegebenenfalls in Wasser und/oder inerten organischen Lösungsmitteln in Anwesenheit einer Base, gegebenenfalls in Gegenwart eines Katalysators umsetzt.

8. Verfahren zur Herstellung von Diarylsulfidderivaten der Formel

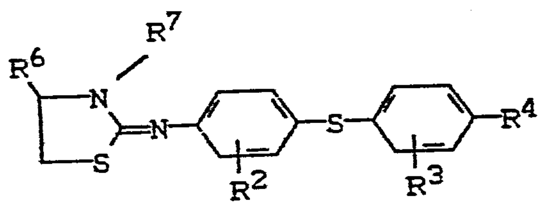

(Id)

in welcher

$R^6$ - für Wasserstoff, Alkyl oder Aryl steht, und
$R^7$ - für Alkyl, Cycloalkyl, Aryl oder Acyl steht,

$R^2$, $R^3$ - gleich oder verschieden sind und
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

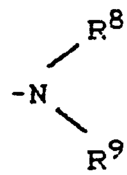

stehen,

worin

$R^8$, $R^9$ - gleich oder verschieden sind und
- für Wasserstoff, Alkyl, Aryl, Aralkyl, Acyl, Trifluoracetyl, Alkylsulfonyl, Phenylsulfonyl, Trifluormethylphenylsulfonyl oder Tolylsulfonyl stehen,

und

$R^4$ - für einen Rest der Formeln

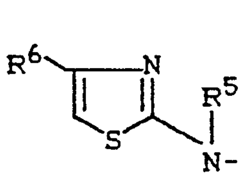

,

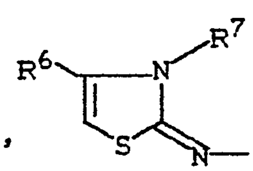

,

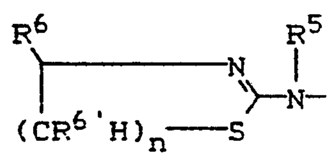

oder

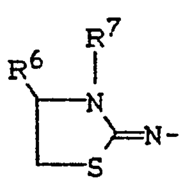

steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

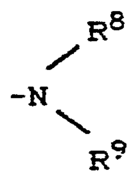

steht,

wobei

$R^8$, $R^9$ die bereits angegebene Bedeutung haben,

dadurch gekennzeichnet, daß man Dihalogenverbindungen der allgemeinen Formel (XIV),

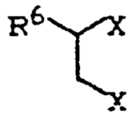

(XIV)

in welcher

$R^6$ - die angegebene Bedeutung hat und
X - für Chlor, Brom oder Iod steht,

mit substituierten Thioharnstoffen der allgemeinen Formel (IX)

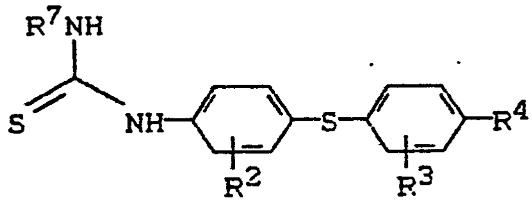

(IX)

in welcher
$R^2$-$R^4$, $R^7$ - die angegebene Bedeutung haben,

gegebenenfalls in Wasser und/oder inerten organischen Lösungsmitteln, gegebenenfalls in Anwesenheit von Basen, umsetzt und gegebenenfalls anfallende Salze mit Basen in die freien Verbindungen überführt.

9. Arzneimittel, enthaltend Diarylsulfidderivate der Formel

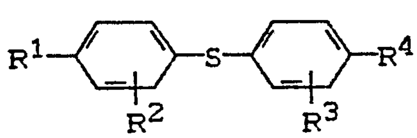

(I),

in welcher

$R^1$ - für einen Thiazolamino-Rest der Formeln

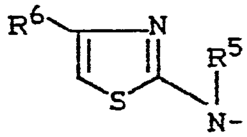 , 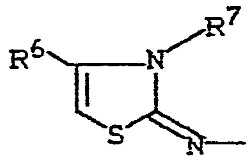 , 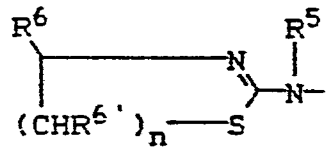

oder 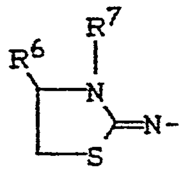 steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

$R^2$, $R^3$ - gleich oder verschieden sind und

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

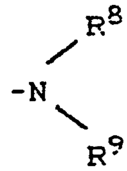 stehen,

worin
$R^8$, $R^9$ - gleich oder verschieden sind und
- für Wasserstoff, Alkyl, Aryl, Aralkyl, Acyl, Trifluoracetyl, Alkylsulfonyl, Arylsulfonyl, Trifluormethylphenylsulfonyl oder Tolylsulfonyl stehen,

und

$R^4$ - eine der oben genannten Bedeutungen von $R^1$ hat,

oder
- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

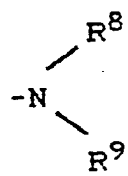 steht,

wobei
$R^8$, $R^9$ die bereits angegebene Bedeutung haben .

und/oder deren Salze.

10. Arzneimittel nach Anspruch 9, enthaltend 0,5 bis 90 Gew.-% des Diarylsulfidderivates.

11. Verwendung von Diarylsulfidderivate der Formel

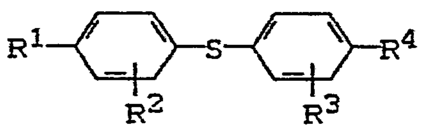

(I),

in welcher

$R^1$ - für einen Thiazolamino-Rest der Formeln

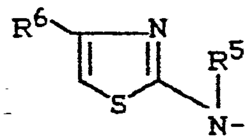

,

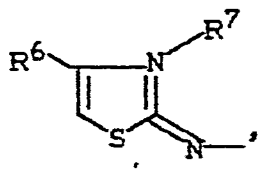

,

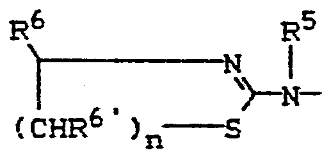

oder

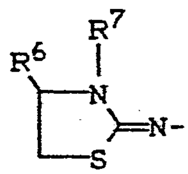

steht,

worin

$R^5$ - für Wasserstoff, Alkyl, Aralkyl oder Acyl steht,

$R^6$, $R^{6'}$ - gleich oder verschieden sind und für Wasserstoff, Alkyl, Aralkyl oder Aryl steht,

$R^7$ - für Alkyl, Cycloalkyl, Aralkyl, Aryl oder Acyl steht,

und

n - für eine Zahl 1 oder 2 steht,

$R^2$, $R^3$ - gleich oder verschieden sind und - für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

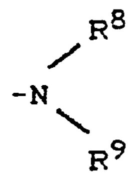

stehen,

worin

$R^8$, $R^9$ - gleich oder verschieden sind und - für Wasserstoff, Alkyl, Aryl, Aralkyl, Acyl, Trifluoracetyl, Alkylsulfonyl, Arylsulfonyl, Trifluormethylphenylsulfonyl oder Tolylsulfonyl stehen,

und

$R^4$ - eine der oben genannten Bedeutungen von $R^1$ hat,

oder

- für Wasserstoff, Alkyl, Alkenyl, Cycloalkyl, Alkoxy, Alkylthio, Halogenalkyl, Halogenalkoxy, Halogenalkylthio, Aryl, Aralkyl, Aryloxy, Aralkoxy, Aralkylthio, Acyl, Carboxy, Alkoxycarbonyl, Carboxyalkyl, Alkoxycarbonylalkyl, Nitro, Cyano, Halogen oder für eine Gruppe der Formel

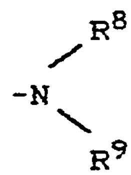 steht,

wobei

$R^8$, $R^9$ die oben angegebene Bedeutung haben

und/oder deren Salze zur Herstellung von Arzneimitteln.

12. Verwendung nach Anspruch 11 zur Herstellung von Lipoxygenasehemmer.

0240680

Europäisches Patentamt

# EUROPÄISCHER RECHERCHENBERICHT

Nummer der Anmeldung

EP 87102529.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | CHEMICAL ABSTRACTS, Band 98, Nr. 3, 17. Jänner 1983, Columbus, Ohio, USA<br>DAMAS, J.; DEFLANDRE, E.; BETHEGNIES, G.; GESQUIERE, J.C. "Prostaglandin synthesis inhibition by 2-[4-(phenylthio) phenylamino]nicotinic acids" Seite 26, Spalte 1, Zusammenfassung-Nr. 11192p<br>& C.R. Seances Soc. Biol. Ses Fil. 1982, 176(4), 558-62<br>-- | 1,9-11 | C 07 D 277/10<br>C 07 D 277/22<br>C 07 D 279/06<br>C 07 D 417/12<br>A 61 K 31/425<br>A 61 K 31/54 |
| A | CHEMICAL ABSTRACTS, Band 94, Nr. 3, 19. Jänner 1981, Columbus, Ohio, USA<br>MARCINCAL-LEFEBVRE, A.; GESQUIERE, J.C.; B. HEGNIES, G.; DUPUIS, B.; VINCENT, A.; LEMER, C. "2-[2-(Phenylthio)phenylamino]nicotinic acids and 2-[4-(phenylthio) phenylamino]nicotinic acids. Synthesis and antiinflammatory activity" Seite 421, Spalte 1, Zusammenfassung-Nr. 15515m<br>& Ann. Pharm. Fr. 1980, 38(3), 243-52<br>---- | 1,5,9-11 | RECHERCHIERTE SACHGEBIETE (Int. Cl. 4)<br>C 07 D 277/00<br>C 07 D 279/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 24-06-1987 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82